# EUROPÄISCHE PATENTSCHRIFT

(11) **EP 3 076 930 B1**
(45) Veröffentlichungstag und Bekanntmachung des Hinweises auf die Patenterteilung: **21.02.2018**
(21) Anmeldenummer: 14799820.7
(22) Anmeldetag: 20.11.2014
(51) Int. Cl.: A61K 8/34, A61K 8/46, A61Q 5/10

(54) **MITTEL ZUM OXIDATIVEN FÄRBEN VON HAAREN ENTHALTEND SPEZIELLE KOMBINATIONEN VON ENTWICKLERN UND KUPPLERN**
HAIR DYEING COMPOSITIONS COMPRISING SPECIFIC KOMBINATIONS OF DEVELOPERS AND COUPLERS
COMPOSITIONS Ä TEINTURE DES CHEVEUX COMPRENNANT DES COMBINAISONS SPECIFIQUES DES DEVELOPERS ET DES COUPLEURS

(30) Priorität: 06.12.2013 DE 102013225195
(43) Veröffentlichungstag der Anmeldung: 12.10.2016
(73) Patentinhaber: Henkel AG & Co. KGaA, 40589 Düsseldorf (DE)
(72) Erfinder: GEBERT-SCHWARZWÄLDER, Antje, 41469 Neuss (DE); KROOS, Astrid, 40789 Monheim (DE)
(86) Internationale Anmeldenummer: PCT/EP2014/075138
(87) Internationale Veröffentlichungsnummer: WO 2015/082227

(56) Entgegenhaltungen:
- EP-A2- 1 166 748
- EP-A2- 1 250 908
- WO-A2-01/41714
- WO-A2-2012/007219

## Beschreibung

Gegenstand der vorliegenden Anmeldung ist ein Mittel zum oxidativen Färben von keratinischen Fasern, insbesondere menschlichen Haaren, welches spezielle Kombinationen von Oxidationsfarbstoffvorprodukten in bestimmten Molverhältnissen zueinander enthält. Das erfindungsgemäße Mittel enthält 4,5-Diamino-1-(2-hydroxyethyl)-1H-pyrazol als ersten Entwickler, mindestens ein Derivat des p-Diaminobenzols als zweiten Entwickler, mindestens ein Derivat des m-Diaminobenzols als ersten Kuppler sowie mindestens ein Derivat des m-Aminophenols als zweiten Kuppler, wobei der erste Entwickler und der zweite Entwickler in einem Molverhältnis von mindestens 1,2 zueinander eingesetzt werden. Weitere Gegenstände der Anmeldung sind eine Mehrkomponenten-Verpackungseinheit umfassend das vorgenannte Mittel sowie die Verwendung des Mittels zur Verbesserung von Lichtechtheit und Waschechtheit von oxidativen Färbungen.

Die Veränderung der Farbe von keratinischen Fasern, insbesondere von Haaren, stellt einen wichtigen Bereich der modernen Kosmetik dar. Hierdurch kann das Erscheinungsbild der Haare sowohl aktuellen Modeströmungen als auch den individuellen Wünschen der einzelnen Person angepasst werden. Zur Farbveränderung der Haare kennt der Fachmann verschiedene Möglichkeiten. Durch den Einsatz von direktziehenden Farbstoffen kann die Haarfarbe temporär verändert werden. Hierbei diffundieren bereits fertig ausgebildete Farbstoffe aus dem Färbemittel in die Haarfaser hinein. Die Färbung mit direktziehenden Farbstoffen ist mit einer geringen Haarschädigung verbunden, ein Nachteil ist jedoch die geringe Haltbarkeit und die schnelle Auswaschbarkeit der mit direktziehenden Farbstoffen erhaltenen Färbungen.

Wünscht sich der Verbraucher ein lang anhaltendes Farbergebnis oder eine Nuance, die heller als seine Ausgangshaarfarbe ist, werden üblicherweise oxidative Farbveränderungsmittel eingesetzt. Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten, die unter dem Einfluss von Oxidationsmitteln untereinander die eigentlichen Farbstoffe ausbilden. Oxidationsfärbemittel zeichnen sich durch lang anhaltende Färbeergebnisse aus.

Zu oxidativen Färbemitteln existiert bereits umfangreicher Stand der Technik. Insbesondere zur Optimierung der Echtheitseigenschaften der mit diesen Mitteln erzielbaren Färbungen wurden schon viele Versuche unternommen.

Doch trotz der großen Anzahl der bereits durchgeführten Optimierungsversuche besteht bei den Echtheitseigenschaften von oxidativ gefärbten Keratinfasern - insbesondere wenn diese in einer Modenuance im Rotbereich gefärbt werden sollen - immer noch Verbesserungsbedarf. Vor allem die Waschechtheiten und Lichtechtheiten von Rot- Violett- und Kupfernuancen können noch nicht als optimal eingestuft werden.

Die Aufgabe der vorliegenden Erfindung war daher die Bereitstellung von oxidativen Färbemitteln zur Erzielung von Rot- Violett- und Kupfernuancen mit verbesserten Waschechtheiten und verbesserten Lichtechtheiten.

Unter der Waschechtheit einer Farbnuance wird die farbliche Veränderung der mit dieser Nuance gefärbten Haarsträhne unter dem Einfluss von mehreren Haarwäschen verstanden. Bei dieser farblichen Veränderung kann es sich sowohl um eine Verschiebung der Farbe in Richtung eines anderen Farbtons als auch um das Verblassen der Färbung handeln. Beide Farbänderungen sind vom Anwender gleichermaßen unerwünscht. Farbnuancen mit guter Waschechtheit verändern sich farblich auch nach wiederholten Haarwäschen nicht oder kaum. Die Haarwäsche kann hierbei unter Zuhilfenahme eines Shampoos, eines konditionierenden Shampoos oder auch eines Conditioners erfolgen. Unter der Lichtechtheit einer Farbnuance wird die farbliche Veränderung der mit dieser Nuance gefärbten Haarsträhne unter dem Einfluss von Tageslicht (d.h. von Sonneneinstrahlung bzw. UV- oder UV/Vis-Strahlung) verstanden. Bei der Bestrahlung mit Tageslicht ist in der Regel ein Verblassen des gefärbten Haares zu beobachten. Nuancen mit guter Lichtechtheit weisen auch nach einer mehrtägigen Bestrahlung mit Sonnenlicht kein sichtbares Verblassen der Färbung auf.

Überraschenderweise hat sich nun herausgestellt, dass auf keratinischen Fasern Färbungen mit hervorragende Waschechtheiten und Lichtechtheiten erzeugt werden können, wenn diese mit Mitteln gefärbt werden, die spezielle Kombinationen aus zwei bestimmten Oxidationsfarbstoffvorprodukten vom Entwicklertyp und zwei bestimmten Oxidationsfarbstoffvorprodukten vom Kupplertyp enthalten und wenn die Oxidationsfarbstoffvorprodukte vom Entwicklertyp in bestimmten Molverhältnissen zueinander eingesetzt werden.

Ein erster Gegenstand der vorliegenden Erfindung ist ein Mittel zum oxidativen Färben von keratinischen Fasern, enthaltend in einem kosmetischen Träger
1. Mittel zum oxidativen Färben von keratinischen Fasern, enthaltend in einem kosmetischen Träger
   (A) als Entwickler 4,5-Diamino-1-(2-hydroxyethyl)-1H-pyrazol und/oder eines seiner physiologisch verträglichen Salze,
   (B) als Entwickler mindestens ein p-Diaminobenzolderivat aus der Gruppe p-Toluylendiamin, 2-(2,5-Diaminophenyl)ethanol und/oder eines seiner physiologisch verträglichen Salze,
   (C) als Kuppler mindestens ein m-Diaminobenzolderivat aus der Gruppe 2-(2,4-Diaminophenoxy)ethanol, 1-Methoxy-2-amino-4-(2'-hydroxyethylamino)benzol, 2,6-Bis(2'-hydroxyethylamino)-1-methylbenzol und/oder eines seiner physiologisch verträglichen Salze und
   (D) als Kuppler mindestens ein m-Aminophenolderivat aus der Gruppe 3-Aminophenol, 5-Amino-2-methylphenol und/oder eines seiner physiologisch verträglichen Salze,
   wobei
   - das molare Verhältnis aus allen im Mittel enthaltenen Entwicklern der Gruppe (A) zu allen im Mittel enthaltenen Entwicklern der Gruppe (B), d.h. das molare Verhältnis (A)/(B), bei einem Wert von mindestens 1,2 liegt.

Unter keratinischen Fasern, keratinhaltigen Fasern oder Keratinfasern sind Pelze, Wolle, Federn und insbesondere menschliche Haare zu verstehen. Obwohl die erfindungsgemäßen Mittel in erster Linie zum Aufhellen und Färben von Keratinfasern geeignet sind, steht prinzipiell einer Verwendung auch auf anderen Gebieten nichts entgegen.

Die Mittel enthalten die erfindungswesentlichen Oxidationsfarbstoffvorprodukte jeweils in einem kosmetischen Träger, bevorzugt in einem geeigneten wässrigen, alkoholischen oder wässrigalkoholischen Träger. Zum Zwecke der oxidativen Farbänderung können solche Träger beispielsweise Cremes, Emulsionen, Gele oder auch tensidhaltige schäumende Lösungen, wie beispielsweise Shampoos, Schaumaerosole, Schaumformulierungen oder andere Zubereitungen, die für die Anwendung auf dem Haar geeignet sind, sein. Besonders bevorzugt handelt es sich bei Mitteln zum oxidativen Färben von keratinischen Fasern um Cremes oder Emulsionen.

Kennzeichnend für die erfindungsgemäßen Mittel ist ihr Gehalt an Oxidationsfarbstoffvorprodukten vom Entwicklertyp (A) und (B), ihr Gehalt an Oxidationsfarbstoffvorprodukten vom Kupplertyp (C) und (D), sowie das bestimmte Molverhältnis der Entwickler (A)/(B) zueinander.

Unter einem Entwickler wird im Sinne der vorliegenden Erfindung ein Oxidationsfarbstoffvorprodukt vom Entwicklertyp verstanden. Unter einem Kuppler wird im Sinne der vorliegenden Erfindung ein Oxidationsfarbstoffvorprodukt vom Kupplertyp verstanden.

Als erstes Oxidationsfarbstoffvorprodukt vom Entwicklertyp (A) enthalten die erfindungsgemäßen Mittel 4,5-Diamino-1-(2-hydroxyethyl)-1H-pyrazol und/oder eines seiner physiologisch verträglichen Salze. Bei 4,5-Diamino-1-(2-hydroxyethyl)-1H-pyrazol handelt es sich um die Verbindung der Formel (I).

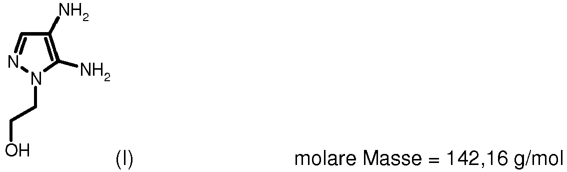

Bevorzugte physiologisch verträgliche Salze von 4,5-Diamino-1-(2-hydroxyethyl)-1H-pyrazol sind insbesondere die Hydrochloride (Monohydrochlorid x HCl, oder Dihydrochlorid x 2 HCl), das Sulfat (x H₂SO₄) und die Hydrobromide (Monohydrobromid x HBr, oder Dihydrobromid x 2 HBr) der Verbindung. Ganz besonders bevorzugt ist 4,5-Diamino-1-(2-hydroxyethyl)-1H-pyrazol Sulfat (Formel (Ia)).

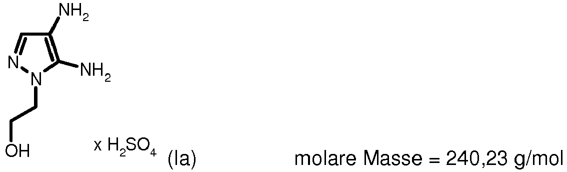

Als zweites Oxidationsfarbstoffvorprodukt vom Entwicklertyp (B) enthalten die erfindungsgemäßen Mittel mindestens ein p-Diaminobenzolderivat aus der Gruppe p-Toluylendiamin, 2-(2,5-Diaminophenyl)ethanol und/oder eines ihrer physiologisch verträglichen Salze.

Bei p-Toluylendiamin handelt es sich um eine Verbindung der Formel (II)

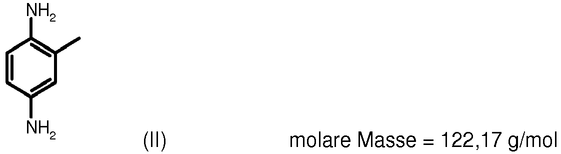

Bevorzugte physiologisch verträgliche Salze von p-Toluylendiamin sind insbesondere die Hydrochloride (Monohydrochlorid x HCl, oder Dihydrochlorid x 2 HCl), das Sulfat (x H₂SO₄) und die Hydrobromide (Monohydrobromid x HBr, oder Dihydrobromid x 2 HBr) der Verbindung. Ganz besonders bevorzugt ist p-Toluylendiamin Sulfat (Formel (IIa)).

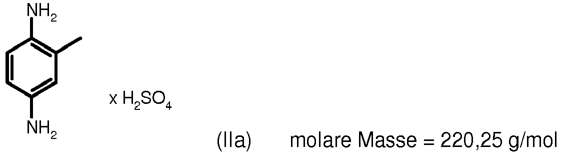

Bei 2-(2,5-Diaminophenyl)ethanol handelt es sich um die Verbindung der Formel (III).

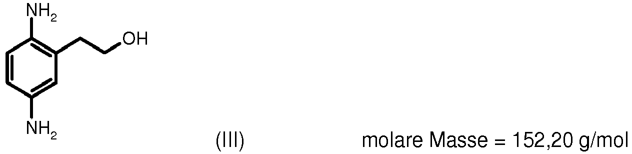

Bevorzugte physiologisch verträgliche Salze von 2-(2,5-Diaminophenyl)ethanol sind insbesondere die Hydrochloride (Monohydrochlorid x HCl, oder Dihydrochlorid x 2 HCl), das Sulfat (x H₂SO₄) und die Hydrobromide (Monohydrobromid x HBr, oder Dihydrobromid x 2 HBr) der Verbindung. Ganz besonders bevorzugt ist 2-(2,5-Diaminophenyl)ethanol Sulfat (Formel (IIIa)).

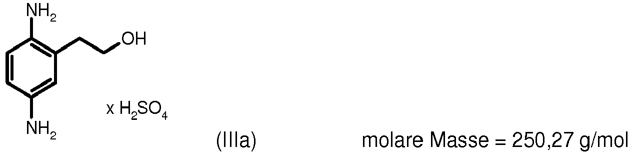

Als erstes Oxidationsfarbstoffvorprodukt vom Kupplertyp (C) enthalten die erfindungsgemäßen Mittel mindestens ein m-Diaminobenzolderivat aus der Gruppe 2-(2,4-Diaminophenoxy)ethanol, 1-Methoxy-2-amino-4-(2'-hydroxyethylamino)benzol, 2,6-Bis(2'-hydroxyethylamino)-1-methylbenzol und/oder eines der physiologisch verträglichen Salze dieser m-Diaminobenzolderivate.

Bei 2-(2,4-Diaminophenoxy)ethanol handelt es sich um die Verbindung der Formel (IV).

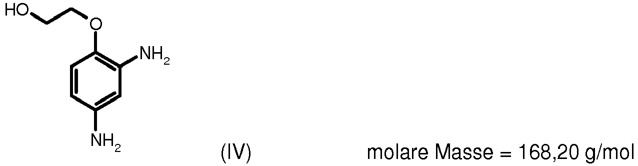

Bevorzugte physiologisch verträgliche Salze von 2-(2,4-Diaminophenoxy)ethanol sind insbesondere die Hydrochloride (Monohydrochlorid x HCl, oder Dihydrochlorid x 2 HCl), das Sulfat (x H₂SO₄), und die Hydrobromide (Monohydrobromid x HBr, oder Dihydrobromid x 2 HBr) der Verbindung. Ganz besonders bevorzugt ist 2-(2,4-Diaminophenoxy)ethanol (Dihydrochlorid) (Formel (IVa).

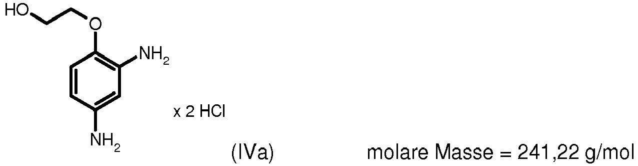

Bei 1-Methoxy-2-amino-4-(2'-hydroxyethylamino)benzol handelt es sich um die Verbindung der Formel (V).

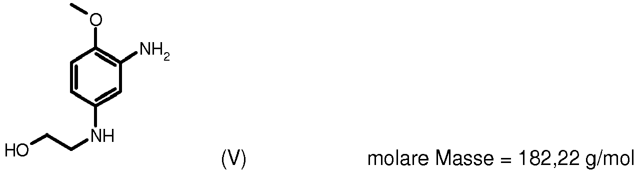

Bevorzugte physiologisch verträgliche Salze von 1-Methoxy-2-amino-4-(2'-hydroxyethylamino)benzol sind insbesondere die Hydrochloride (Monohydrochlorid x HCl, oder Dihydrochlorid x 2 HCl), das Sulfat (x H₂SO₄) und die Hydrobromide (Monohydrobromid x HBr, oder Dihydrobromid x 2 HBr) der Verbindung. Besonders bevorzugt wird 2,6-Bis(2'-hydroxyethylamino)-1-methylbenzol jedoch nicht in Salzform, sondern in Form der freien Verbindung (d.h. als Verbindung der Formel (V)) eingesetzt.

Bei 2,6-Bis(2'-hydroxyethylamino)-1-methylbenzol handelt es sich um die Verbindung der Formel (VI).

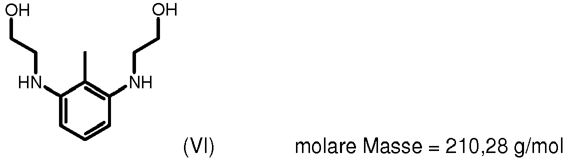

Bevorzugte physiologisch verträgliche Salze von 2,6-Bis(2'-hydroxyethylamino)-1-methylbenzol sind insbesondere die Hydrochloride (Monohydrochlorid x HCl, oder Dihydrochlorid x 2 HCl), das Sulfat (x H₂SO₄) und die Hydrobromide (Monohydrobromid x HBr, oder Dihydrobromid x 2 HBr) der Verbindung. Besonders bevorzugt wird 2,6-Bis(2'-hydroxyethylamino)-1-methylbenzol jedoch nicht in Salzform, sondern in Form der freien Verbindung (d.h. als Verbindung der Formel (VI)) eingesetzt.

Als zweites Oxidationsfarbstoffvorprodukt vom Kupplertyp (D) enthalten die erfindungsgemäßen Mittel mindestens ein m-Aminophenolderivat aus der Gruppe 3-Aminophenol selbst, 5-Amino-2-methylphenol und/oder einem der physiologisch verträglichen Salze diese m-Aminophenolderivate.

Bei 3-Aminophenol handelt es sich um die Verbindung der Formel (VII).

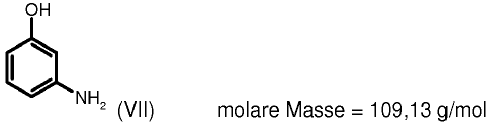

Bevorzugte physiologisch verträgliche Salze von 3-Aminophenol sind insbesondere die Hydrochloride (Monohydrochlorid x HCl, oder Dihydrochlorid x 2 HCl), das Sulfat (x H₂SO₄) und die Hydrobromide (Monohydrobromid x HBr, oder Dihydrobromid x 2 HBr) der Verbindung. Besonders bevorzugt wird 3-Aminophenol jedoch nicht in Salzform, sondern in Form der freien Verbindung (d.h. als Verbindung der Formel (VII)) eingesetzt.

Bei 5-Amino-2-methylphenol handelt es sich um die Verbindung der Formel (VIII). Ein Alternativname für 5-Amino-2-methylphenol ist auch 4-Amino-2-hydroxytoluol.

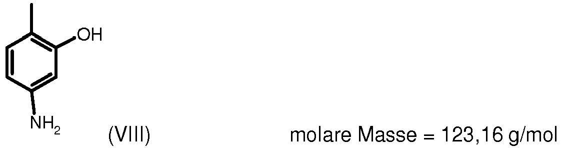

Bevorzugte physiologisch verträgliche Salze von 5-Amino-2-methylphenol sind insbesondere die Hydrochloride (Monohydrochlorid x HCl, oder Dihydrochlorid x 2 HCl), das Sulfat (x H₂SO₄) und die Hydrobromide (Monohydrobromid x HBr, oder Dihydrobromid x 2 HBr) der Verbindung. Besonders bevorzugt wird 5-Amino-2-methylphenol nicht in Salzform, sondern in Form der freien Verbindung (d.h. als Verbindung der Formel (VIII)) eingesetzt.

Im Zuge der zu dieser Erfindung führenden Arbeiten hat sich herausgestellt, dass der Einsatz einer Kombination der vier verschiedenen Oxidationsfarbstoffvorprodukte (A), (B), (C) und (D) im Vergleich zu einem Einsatz von nur drei Oxidationsfarbstoffvorprodukten (wie z.B. von (A), (C) und (D), oder von (A), (B) und (C)) in oxidativen Färbemitteln zu einer Verbesserung der Waschechtheit und zusätzlich auch zu einer Verbesserung der Lichtechtheit führt, wenn die Oxidationsfarbstoffe vom Entwicklertyp (A) und (B) in einem bestimmten Molverhältnis (A)/(B) zueinander eingesetzt werden.

Die Molmasse (Einheit g/mol) einer Verbindung ist definiert als Masse (Einheit g) pro Stoffmenge (Einheit Mol).

Während des oxidativen Färbeprozesses reagiert jeweils ein Molekül eines Oxidationsfarbstoffvorprodukts vom Entwicklertyp mit einem oder mehreren Molekülen der Oxidationsfarbstoffvorprodukte vom Kupplertyp. Es hat sich herausgestellt, dass hohe Farbintensitäten und gute Echtheitseigenschaften erzielt werden können, wenn die Stoffmengen der miteinander reagierenden Farbstoffvorprodukte (d.h. ihre Molmengen) in optimaler Weise aufeinander abgestimmt werden.

Unter der Stoffmenge wird die Quantität einer Stoffportion auf der Grundlage der Anzahl der jeweils darin enthalten Teilchen verstanden. Die Einheit der Stoffmenge ist die Basiseinheit Mol (mol bzw. mmol).

Die Molmenge des in einem Färbemittel enthaltenen Oxidationsfarbstoffvorproduktes kann durch Division der Einsatzmenge durch seine molare Menge erhalten werden. Beispiel:
100 g einer Färbecreme enthalten
(A) 4,80 g 4,5-Diamino-1-(2-hydroxyethyl)-1H-pyrazol Sulfat
molare Masse (4,5-Diamino-1-(2-hydroxyethyl)-1H-pyrazol Sulfat) = 240,23 g/mol

Die Molmenge des im Mittel (100 g) enthaltenen (4,5-Diamino-1-(2-hydroxyethyl)-1H-pyrazol Sulfat) beträgt (4,80 g / [240,23 g/mol) = 0,020 mol (entsprechend 20,0 mmol).

Es hat sich nun herausgestellt, dass die Formulierung von Nuancen im Rot-, Kupfer bzw. Violettbereich möglich wird, wenn den Entwicklern der Gruppe (A) ein Unterschuss an Entwicklern der Gruppe (B) beigemischt wird. Überraschenderweise lassen sich auf diese Weise auch die Lichtechtheiten und Waschechtheiten der mit diesen Nuancen erzielbaren Färbungen verbessern.

Vorteilhafte Auswirkungen auf die Lichtechtheit und Waschechtheit der mit diesen Mitteln erzielbaren Färbungen können demnach dann beobachtet werden, wenn in den Mitteln die Entwickler aus der Gruppe (A) in einem molaren Überschuss zu den Entwicklern aus der Gruppe (B) eingesetzt wurden, d.h. wenn das molare Verhältnis (A)/(B) bei einem Wert von größer als 1,2 liegt.

Die Berechnungsgrundlage für die Berechnung des molaren Verhältnisses (A)/(B) ist die molare Gesamtmenge aller im Mittel enthaltenen Entwickler der Gruppe (A) (d.h. die Molmenge von 4,5-Diamino-1-(2-hydroxyethyl)-1H-pyrazol zuzüglich der Molmengen von dessen Salzen), die zur molaren Gesamtmenge aller im Mittel enthaltenen Entwicklern der Gruppe (B) (d.h. der Molmenge von p-Toluylendiamin und 2-(2,5-Diaminophenyl)ethanol zuzüglich der Molmengen von deren Salzen) in Relation gesetzt wird. Hierbei muss aus der Gruppe der Entwickler (B) mindestens eine Verbindung erfindungsgemäße Verbindung enthalten sein.

Ein ganz besonders bevorzugtes Mittel zum oxidativen Färben von keratinischen Fasern ist dadurch gekennzeichnet, dass das molare Verhältnis aus allen im Mittel enthaltenen Entwicklern der Gruppe (A) zu allen im Mittel enthaltenen Entwicklern der Gruppe (B), d.h. das molare Verhältnis (A)/(B), bei einem Wert von mindestens 1,4, bevorzugt von mindestens 1,6, weiter bevorzugt von mindestens 1,8 und besonders bevorzugt von mindestens 1,9 liegt.

Die besten Waschechtheiten und die besten Lichtechtheiten konnten erzielt werden konnten, wenn die erfindungsgemäßen Mittel die Entwickler der Gruppe (A) in ungefähr doppelt so großer Gesamtmolmenge wie die Entwickler aus der Gruppe (B) enthielten.

Damit wiesen die oxidativen Färbemittel, welche die Entwickler aus der Gruppe (A) und die Entwickler aus der Gruppe (B) in einem molaren Verhältnis von 1,8 bis 2,5 enthielten, die besten Echtheitseigenschaften auf. Steigt das molare Verhältnis von (A)/(B) dagegen noch weiter auf Werte von über 4,5 so zeigte sich wieder eine Tendenz zur Verschlechterung der Waschechtheit und der Lichtechtheit der mit diesen Mitteln erhaltenen Färbungen. Aus diesem Grund ist es von Vorteil, wenn das molare Verhältnis (A)/(B) Werte von 4,5 nicht übersteigt.

Ein ganz besonders bevorzugtes Mittel oxidativen Färben von keratinischen Fasern ist dadurch gekennzeichnet, dass das molare Verhältnis aus allen im Mittel enthaltenen Entwicklern der Gruppe (A) zu allen im Mittel enthaltenen Entwicklern der Gruppe (B), d.h. das molare Verhältnis (A)/(B), bei einem Wert von maximal 4,5, bevorzugt von maximal 4,0, weiter bevorzugt von maximal 3,5, noch weiter bevorzugt von maximal 3,0 und besonders bevorzugt von maximal 2,5 liegt.

### Beispiel:

100 g einer Färbecreme enthalten
- (A) 4,80 g 4,5-Diamino-1-(2-hydroxyethyl)-1H-pyrazol Sulfat
   Die Molmenge des im Mittel enthaltenen (4,5-Diamino-1-(2-hydroxyethyl)-1H-pyrazol Sulfat) beträgt (4,80 g / [240,23 g/mol]) = 0,020 mol (entsprechend 20,0 mmol)
- (B) 2,50 g 2-(2,5-Diaminophenyl)ethanol Sulfat
   Die Molmenge des im Mittel enthaltenen 2-(2,5-Diaminophenyl)ethanol Sulfat beträgt (2,50 g / [250,23 g/mol]) = 0,010 mol (entsprechend 10,0 mmol).

Das molare Verhältnis (A)/(B) liegt bei einem Wert von (0,020 mol) / (0,010 mol) = 2.

Als zweites Oxidationsfarbstoffvorprodukt vom Entwicklertyp (B) enthalten die erfindungsgemäßen Mittel mindestens ein p-Diaminobenzolderivat aus der Gruppe p-Toluylendiamin, 2-(2,5-Diaminophenyl)ethanol und/oder eines ihrer physiologisch verträglichen Salze.

Beide Entwickler aus der Gruppe (B) haben sich in Kombination mit den weiteren erfindungsgemäßen Oxidationsfarbstoffvorprodukten (A), (C) und (D) als hervorragend geeignet zur Erzielung von Färbungen mit guter Lichtechtheit und Waschechtheit herausgestellt.

Insbesondere die Beimischung von p-Toluylendiamin führte in den vorgenannten Kombinationen und molaren Verhältnissen zu Färbungen mit herausragenden Echtheitseigenschaften.

In einer weiteren ganz besonders bevorzugten Ausführungsform ist ein erfindungsgemäßes Mittel zum oxidativen Färben von keratinischen Fasern daher dadurch gekennzeichnet, dass es
(B) als Entwickler p-Toluylendiamin und/oder eines seiner physiologisch verträglichen Salze enthält.

Der Entwickler p-Toluylendiamin bzw. seine Salze können unter Umständen jedoch bei besonders empfindlichen Anwendern ein sensibilisierendes Potential aufweisen, daher gibt es aus toxikologischen Gründen zunehmende Bestrebungen, die Substanz p-Toluylendiamin in oxidativen Färbemitteln zu substituieren.

Hierbei hat sich herausgestellt, dass (B) 2-(2,5-Diaminophenyl)ethanol bei Einsatz in Kombination mit dem Entwickler (A) und den Kupplern (C) und (D) ähnlich gute Echtheitsresultate liefert. Darüber hinaus ist 2-(2,5-Diaminophenyl)ethanol auch aus toxikologischen Gründen stark bevorzugt.

In einer weiteren ganz besonders bevorzugten Ausführungsform ist ein erfindungsgemäßes Mittel zum oxidativen Färben von keratinischen Fasern daher dadurch gekennzeichnet, dass es
(B) als Entwickler 2-(2,5-Diaminophenyl)ethanol und/oder eines seiner physiologisch verträglichen Salze enthält.

Als erstes Oxidationsfarbstoffvorprodukt vom Kupplertyp (C) enthalten die erfindungsgemäßen Mittel mindestens ein m-Diaminobenzolderivat aus der Gruppe 2-(2,4-Diaminophenoxy)ethanol, 1-Methoxy-2-amino-4-(2'-hydroxyethylamino)benzol, 2,6-Bis(2'-hydroxyethylamino)-1-methylbenzol und/oder eines der physiologisch verträglichen Salze dieser m-Diaminobenzolderivate.

Im Hinblick auf die Verbesserung der Echtheitseigenschaften haben sich insbesondere 2-(2,4-Diaminophenoxy)ethanol und 2,6-Bis(2'-hydroxyethylamino)-1-methylbenzol als ganz besonders geeignet erwiesen.

In einer weiteren ganz besonders bevorzugten Ausführungsform ist ein erfindungsgemäßes Mittel zum oxidativen Färben von keratinischen Fasern daher dadurch gekennzeichnet, dass es
(C) als Kuppler 2-(2,4-Diaminophenoxy)ethanol und/oder eines seiner physiologisch verträglichen Salze enthält.

In einer weiteren ganz besonders bevorzugten Ausführungsform ist ein erfindungsgemäßes Mittel zum oxidativen Färben von keratinischen Fasern daher dadurch gekennzeichnet, dass es
(C) als Kuppler 2,6-Bis(2'-hydroxyethylamino)-1-methylbenzol und/oder eines seiner physiologisch verträglichen Salze enthält.

Als zweites Oxidationsfarbstoffvorprodukt vom Kupplertyp (D) enthalten die erfindungsgemäßen Mittel mindestens ein m-Aminophenolderivat aus der Gruppe 3-Aminophenol, 5-Amino-2-methylphenol und/oder eines der physiologisch verträglichen Salze dieser m-Aminophenolderivate.

Im Hinblick auf die Optimierung der Echtheitseigenschaften hat sich der der Kuppler 5-Amino-2-methylphenol aus der Gruppe (D) als ganz besonders geeignet erwiesen.

In einer weiteren ganz besonders bevorzugten Ausführungsform ist ein erfindungsgemäßes Mittel zum oxidativen Färben von keratinischen Fasern daher dadurch gekennzeichnet, dass es
(D) als Kuppler 5-Amino-2-methylphenol und/oder eines seiner physiologisch verträglichen Salze enthält.

Besonders bevorzugt ist demnach ein Mittel zum oxidativen Färben von keratinischen Fasern, enthaltend in einem kosmetischen Träger
(A) als Entwickler 4,5-Diamino-1-(2-hydroxyethyl)-1H-pyrazol und/oder eines seiner physiologisch verträglichen Salze,
(B) als Entwickler p-Toluylendiamin und/oder eines seiner physiologisch verträglichen Salze,
(C) als Kuppler 2-(2,4-Diaminophenoxy)ethanol und/oder eines seiner physiologisch verträglichen Salze und
(D) als Kuppler 5-Amino-2-methylphenol und/oder eines seiner physiologisch verträglichen Salze,
   - das molare Verhältnis aus allen im Mittel enthaltenen Entwicklern der Gruppe (A) zu allen im Mittel enthaltenen Entwicklern der Gruppe (B), d.h. das molare Verhältnis (A)/(B), bei einem Wert von mindestens 1,2 liegt.

Besonders bevorzugt ist demnach ein Mittel zum oxidativen Färben von keratinischen Fasern, enthaltend in einem kosmetischen Träger
(A) als Entwickler 4,5-Diamino-1-(2-hydroxyethyl)-1H-pyrazol und/oder eines seiner physiologisch verträglichen Salze,
(B) als Entwickler 2-(2,5-Diaminophenyl)ethanol und/oder eines seiner physiologisch verträglichen Salze,
(C) als Kuppler 2-(2,4-Diaminophenoxy)ethanol und/oder eines seiner physiologisch verträglichen Salze und
(D) als Kuppler 5-Amino-2-methylphenol und/oder eines seiner physiologisch verträglichen Salze,

- das molare Verhältnis aus allen im Mittel enthaltenen Entwicklern der Gruppe (A) zu allen im Mittel enthaltenen Entwicklern der Gruppe (B), d.h. das molare Verhältnis (A)/(B), bei einem Wert von mindestens 1,2 liegt.

Besonders bevorzugt ist demnach ein Mittel zum oxidativen Färben von keratinischen Fasern, enthaltend in einem kosmetischen Träger
(A) als Entwickler 4,5-Diamino-1-(2-hydroxyethyl)-1H-pyrazol und/oder eines seiner physiologisch verträglichen Salze,
(B) als Entwickler p-Toluylendiamin und/oder eines seiner physiologisch verträglichen Salze,
(C) als Kuppler 1-Methoxy-2-amino-4-(2'-hydroxyethylamino)benzol und/oder eines seiner physiologisch verträglichen Salze und
(D) als Kuppler 5-Amino-2-methylphenol und/oder eines seiner physiologisch verträglichen Salze,
   - das molare Verhältnis aus allen im Mittel enthaltenen Entwicklern der Gruppe (A) zu allen im Mittel enthaltenen Entwicklern der Gruppe (B), d.h. das molare Verhältnis (A)/(B), bei einem Wert von mindestens 1,2 liegt.

Besonders bevorzugt ist demnach ein Mittel zum oxidativen Färben von keratinischen Fasern, enthaltend in einem kosmetischen Träger
(A) als Entwickler 4,5-Diamino-1-(2-hydroxyethyl)-1H-pyrazol und/oder eines seiner physiologisch verträglichen Salze,
(B) als Entwickler 2-(2,5-Diaminophenyl)ethanol und/oder eines seiner physiologisch verträglichen Salze,
(C) als Kuppler 1-Methoxy-2-amino-4-(2'-hydroxyethylamino)benzol und/oder eines seiner physiologisch verträglichen Salze und
(D) als Kuppler 5-Amino-2-methylphenol und/oder eines seiner physiologisch verträglichen Salze,
   - das molare Verhältnis aus allen im Mittel enthaltenen Entwicklern der Gruppe (A) zu allen im Mittel enthaltenen Entwicklern der Gruppe (B), d.h. das molare Verhältnis (A)/(B), bei einem Wert von mindestens 1,2 liegt.

Besonders bevorzugt ist demnach ein Mittel zum oxidativen Färben von keratinischen Fasern, enthaltend in einem kosmetischen Träger
(A) als Entwickler 4,5-Diamino-1-(2-hydroxyethyl)-1H-pyrazol und/oder eines seiner physiologisch verträglichen Salze,
(B) als Entwickler p-Toluylendiamin und/oder eines seiner physiologisch verträglichen Salze,
(C) als Kuppler 2,6-Bis(2'-hydroxyethylamino)-1-methylbenzol und/oder eines seiner physiologisch verträglichen Salze und
(D) als Kuppler 5-Amino-2-methylphenol und/oder eines seiner physiologisch verträglichen Salze,
   - das molare Verhältnis aus allen im Mittel enthaltenen Entwicklern der Gruppe (A) zu allen im Mittel enthaltenen Entwicklern der Gruppe (B), d.h. das molare Verhältnis (A)/(B), bei einem Wert von mindestens 1,2 liegt.

Besonders bevorzugt ist demnach ein Mittel zum oxidativen Färben von keratinischen Fasern, enthaltend in einem kosmetischen Träger
(A) als Entwickler 4,5-Diamino-1-(2-hydroxyethyl)-1H-pyrazol und/oder eines seiner physiologisch verträglichen Salze,
(B) als Entwickler 2-(2,5-Diaminophenyl)ethanol und/oder eines seiner physiologisch verträglichen Salze,
(C) als Kuppler 2,6-Bis(2'-hydroxyethylamino)-1-methylbenzol und/oder eines seiner physiologisch verträglichen Salze und
(D) als Kuppler 5-Amino-2-methylphenol und/oder eines seiner physiologisch verträglichen Salze,
   - das molare Verhältnis aus allen im Mittel enthaltenen Entwicklern der Gruppe (A) zu allen im Mittel enthaltenen Entwicklern der Gruppe (B), d.h. das molare Verhältnis (A)/(B), bei einem Wert von mindestens 1,2 liegt.

Besonders bevorzugt ist weiterhin ein Mittel zum oxidativen Färben von keratinischen Fasern, enthaltend in einem kosmetischen Träger
(A) als Entwickler 4,5-Diamino-1-(2-hydroxyethyl)-1H-pyrazol und/oder eines seiner physiologisch verträglichen Salze,
(B) als Entwickler mindestens ein p-Diaminobenzolderivat aus der Gruppe p-Toluylendiamin, 2-(2,5-Diaminophenyl)ethanol und/oder eines seiner physiologisch verträglichen Salze,
(C) als Kuppler mindestens ein m-Diaminobenzolderivat aus der Gruppe 2-(2,4-Diaminophenoxy)ethanol, 1-Methoxy-2-amino-4-(2'-hydroxyethylamino)benzol, 2,6-Bis(2'-hydroxyethylamino)-1-methylbenzol und/oder eines seiner physiologisch verträglichen Salze und
(D) als Kuppler mindestens ein m-Aminophenolderivat aus der Gruppe 3-Aminophenol, 5-Amino-2-methylphenol und/oder eines seiner physiologisch verträglichen Salze,
   wobei
   - das molare Verhältnis aus allen im Mittel enthaltenen Entwicklern der Gruppe (A) zu allen im Mittel enthaltenen Entwicklern der Gruppe (B), d.h. das molare Verhältnis (A)/(B), bei einem Wert von mindestens 1,4 liegt.

Besonders bevorzugt ist weiterhin ein Mittel zum oxidativen Färben von keratinischen Fasern, enthaltend in einem kosmetischen Träger
(A) als Entwickler 4,5-Diamino-1-(2-hydroxyethyl)-1H-pyrazol und/oder eines seiner physiologisch verträglichen Salze,
(B) als Entwickler mindestens ein p-Diaminobenzolderivat aus der Gruppe p-Toluylendiamin, 2-(2,5-Diaminophenyl)ethanol und/oder eines seiner physiologisch verträglichen Salze,
(C) als Kuppler mindestens ein m-Diaminobenzolderivat aus der Gruppe 2-(2,4-Diaminophenoxy)ethanol, 1-Methoxy-2-amino-4-(2'-hydroxyethylamino)benzol, 2,6-Bis(2'-hydroxyethylamino)-1-methylbenzol und/oder eines seiner physiologisch verträglichen Salze und
(D) als Kuppler mindestens ein m-Aminophenolderivat aus der Gruppe 3-Aminophenol, 5-Amino-2-methylphenol und/oder eines seiner physiologisch verträglichen Salze,
wobei
- das molare Verhältnis aus allen im Mittel enthaltenen Entwicklern der Gruppe (A) zu allen im Mittel enthaltenen Entwicklern der Gruppe (B), d.h. das molare Verhältnis (A)/(B), bei einem Wert von mindestens 1,6 liegt.

Besonders bevorzugt ist weiterhin ein Mittel zum oxidativen Färben von keratinischen Fasern, enthaltend in einem kosmetischen Träger
(A) als Entwickler 4,5-Diamino-1-(2-hydroxyethyl)-1H-pyrazol und/oder eines seiner physiologisch verträglichen Salze,
(B) als Entwickler mindestens ein p-Diaminobenzolderivat aus der Gruppe p-Toluylendiamin, 2-(2,5-Diaminophenyl)ethanol und/oder eines seiner physiologisch verträglichen Salze,
(C) als Kuppler mindestens ein m-Diaminobenzolderivat aus der Gruppe 2-(2,4-Diaminophenoxy)ethanol, 1-Methoxy-2-amino-4-(2'-hydroxyethylamino)benzol, 2,6-Bis(2'-hydroxyethylamino)-1-methylbenzol und/oder eines seiner physiologisch verträglichen Salze und
(D) als Kuppler mindestens ein m-Aminophenolderivat aus der Gruppe 3-Aminophenol, 5-Amino-2-methylphenol und/oder eines seiner physiologisch verträglichen Salze,
   wobei
   - das molare Verhältnis aus allen im Mittel enthaltenen Entwicklern der Gruppe (A) zu allen im Mittel enthaltenen Entwicklern der Gruppe (B), d.h. das molare Verhältnis (A)/(B), bei einem Wert von mindestens 1,8 liegt.

Besonders bevorzugt ist weiterhin ein Mittel zum oxidativen Färben von keratinischen Fasern, enthaltend in einem kosmetischen Träger
(A) als Entwickler 4,5-Diamino-1-(2-hydroxyethyl)-1H-pyrazol und/oder eines seiner physiologisch verträglichen Salze,
(B) als Entwickler mindestens ein p-Diaminobenzolderivat aus der Gruppe p-Toluylendiamin, 2-(2,5-Diaminophenyl)ethanol und/oder eines seiner physiologisch verträglichen Salze,
(C) als Kuppler mindestens ein m-Diaminobenzolderivat aus der Gruppe 2-(2,4-Diaminophenoxy)ethanol, 1-Methoxy-2-amino-4-(2'-hydroxyethylamino)benzol, 2,6-Bis(2'-hydroxyethylamino)-1-methylbenzol und/oder eines seiner physiologisch verträglichen Salze und
(D) als Kuppler mindestens ein m-Aminophenolderivat aus der Gruppe 3-Aminophenol, 5-Amino-2-methylphenol und/oder eines seiner physiologisch verträglichen Salze,
   wobei
   - das molare Verhältnis aus allen im Mittel enthaltenen Entwicklern der Gruppe (A) zu allen im Mittel enthaltenen Entwicklern der Gruppe (B), d.h. das molare Verhältnis (A)/(B), bei einem Wert von mindestens 1,9 liegt.

Besonders bevorzugt ist weiterhin ein Mittel zum oxidativen Färben von keratinischen Fasern, enthaltend in einem kosmetischen Träger
(A) als Entwickler 4,5-Diamino-1-(2-hydroxyethyl)-1H-pyrazol und/oder eines seiner physiologisch verträglichen Salze,
(B) als Entwickler mindestens ein p-Diaminobenzolderivat aus der Gruppe p-Toluylendiamin, 2-(2,5-Diaminophenyl)ethanol und/oder eines seiner physiologisch verträglichen Salze,
(C) als Kuppler mindestens ein m-Diaminobenzolderivat aus der Gruppe 2-(2,4-Diaminophenoxy)ethanol, 1-Methoxy-2-amino-4-(2'-hydroxyethylamino)benzol, 2,6-Bis(2'-hydroxyethylamino)-1-methylbenzol und/oder eines seiner physiologisch verträglichen Salze und
(D) als Kuppler mindestens ein m-Aminophenolderivat aus der Gruppe 3-Aminophenol, 5-Amino-2-methylphenol und/oder eines seiner physiologisch verträglichen Salze,
   wobei
   - das molare Verhältnis aus allen im Mittel enthaltenen Entwicklern der Gruppe (A) zu allen im Mittel enthaltenen Entwicklern der Gruppe (B), d.h. das molare Verhältnis (A)/(B), bei einem Wert von 1,4 bis 4,5 liegt.

Besonders bevorzugt ist demnach ein Mittel zum oxidativen Färben von keratinischen Fasern, enthaltend in einem kosmetischen Träger
(A) als Entwickler 4,5-Diamino-1-(2-hydroxyethyl)-1H-pyrazol und/oder eines seiner physiologisch verträglichen Salze,
(B) als Entwickler mindestens ein p-Diaminobenzolderivat aus der Gruppe p-Toluylendiamin, 2-(2,5-Diaminophenyl)ethanol und/oder eines seiner physiologisch verträglichen Salze,
(C) als Kuppler mindestens ein m-Diaminobenzolderivat aus der Gruppe 2-(2,4-Diaminophenoxy)ethanol, 1-Methoxy-2-amino-4-(2'-hydroxyethylamino)benzol, 2,6-Bis(2'-hydroxyethylamino)-1-methylbenzol und/oder eines seiner physiologisch verträglichen Salze und
(D) als Kuppler mindestens ein m-Aminophenolderivat aus der Gruppe 3-Aminophenol, 5-Amino-2-methylphenol und/oder eines seiner physiologisch verträglichen Salze,
   wobei
   - das molare Verhältnis aus allen im Mittel enthaltenen Entwicklern der Gruppe (A) zu allen im Mittel enthaltenen Entwicklern der Gruppe (B), d.h. das molare Verhältnis (A)/(B), bei einem Wert von 1,6 bis 4,0 liegt.

Besonders bevorzugt ist demnach ein Mittel zum oxidativen Färben von keratinischen Fasern, enthaltend in einem kosmetischen Träger
(A) als Entwickler 4,5-Diamino-1-(2-hydroxyethyl)-1H-pyrazol und/oder eines seiner physiologisch verträglichen Salze,
(B) als Entwickler mindestens ein p-Diaminobenzolderivat aus der Gruppe p-Toluylendiamin, 2-(2,5-Diaminophenyl)ethanol und/oder eines seiner physiologisch verträglichen Salze,
(C) als Kuppler mindestens ein m-Diaminobenzolderivat aus der Gruppe 2-(2,4-Diaminophenoxy)ethanol, 1-Methoxy-2-amino-4-(2'-hydroxyethylamino)benzol, 2,6-Bis(2'-hydroxyethylamino)-1-methylbenzol und/oder eines seiner physiologisch verträglichen Salze und
(D) als Kuppler mindestens ein m-Aminophenolderivat aus der Gruppe 3-Aminophenol, 5-Amino-2-methylphenol und/oder eines seiner physiologisch verträglichen Salze,
   wobei
   - das molare Verhältnis aus allen im Mittel enthaltenen Entwicklern der Gruppe (A) zu allen im Mittel enthaltenen Entwicklern der Gruppe (B), d.h. das molare Verhältnis (A)/(B), bei einem Wert von 1,8 bis 3,5 liegt.

Besonders bevorzugt ist demnach ein Mittel zum oxidativen Färben von keratinischen Fasern, enthaltend in einem kosmetischen Träger
(A) als Entwickler 4,5-Diamino-1-(2-hydroxyethyl)-1H-pyrazol und/oder eines seiner physiologisch verträglichen Salze,
(B) als Entwickler mindestens ein p-Diaminobenzolderivat aus der Gruppe p-Toluylendiamin, 2-(2,5-Diaminophenyl)ethanol und/oder eines seiner physiologisch verträglichen Salze,
(C) als Kuppler mindestens ein m-Diaminobenzolderivat aus der Gruppe 2-(2,4-Diaminophenoxy)ethanol, 1-Methoxy-2-amino-4-(2'-hydroxyethylamino)benzol, 2,6-Bis(2'-hydroxyethylamino)-1-methylbenzol und/oder eines seiner physiologisch verträglichen Salze und
(D) als Kuppler mindestens ein m-Aminophenolderivat aus der Gruppe 3-Aminophenol, 5-Amino-2-methylphenol und/oder eines seiner physiologisch verträglichen Salze,
   wobei
   - das molare Verhältnis aus allen im Mittel enthaltenen Entwicklern der Gruppe (A) zu allen im Mittel enthaltenen Entwicklern der Gruppe (B), d.h. das molare Verhältnis (A)/(B), bei einem Wert von 1,9 bis 2,5 liegt.

Besonders bevorzugt ist demnach ein Mittel zum oxidativen Färben von keratinischen Fasern, enthaltend in einem kosmetischen Träger
(A) als Entwickler 4,5-Diamino-1-(2-hydroxyethyl)-1H-pyrazol und/oder eines seiner physiologisch verträglichen Salze,
(B) als Entwickler p-Toluylendiamin und/oder eines seiner physiologisch verträglichen Salze,
(C) als Kuppler 2-(2,4-Diaminophenoxy)ethanol und/oder eines seiner physiologisch verträglichen Salze und
(D) als Kuppler 5-Amino-2-methylphenol und/oder eines seiner physiologisch verträglichen Salze
   wobei
   - das molare Verhältnis aus allen im Mittel enthaltenen Entwicklern der Gruppe (A) zu allen im Mittel enthaltenen Entwicklern der Gruppe (B), d.h. das molare Verhältnis (A)/(B), bei einem Wert von 1,9 bis 2,5 liegt.

Besonders bevorzugt ist demnach ein Mittel zum oxidativen Färben von keratinischen Fasern, enthaltend in einem kosmetischen Träger
(A) als Entwickler 4,5-Diamino-1-(2-hydroxyethyl)-1H-pyrazol und/oder eines seiner physiologisch verträglichen Salze,
(B) als Entwickler 2-(2,5-Diaminophenyl)ethanol und/oder eines seiner physiologisch verträglichen Salze,
   und/oder eines seiner physiologisch verträglichen Salze,
(C) als Kuppler 2-(2,4-Diaminophenoxy)ethanol und/oder eines seiner physiologisch verträglichen Salze und
(D) als Kuppler 5-Amino-2-methylphenol und/oder eines seiner physiologisch verträglichen Salze
   wobei
   - das molare Verhältnis aus allen im Mittel enthaltenen Entwicklern der Gruppe (A) zu allen im Mittel enthaltenen Entwicklern der Gruppe (B), d.h. das molare Verhältnis (A)/(B), bei einem Wert von 1,9 bis 2,5 liegt.

Besonders bevorzugt ist demnach ein Mittel zum oxidativen Färben von keratinischen Fasern, enthaltend in einem kosmetischen Träger
(A) als Entwickler 4,5-Diamino-1-(2-hydroxyethyl)-1H-pyrazol und/oder eines seiner physiologisch verträglichen Salze,
(B) als Entwickler p-Toluylendiamin und/oder eines seiner physiologisch verträglichen Salze,
(C) als Kuppler 2,6-Bis(2'-hydroxyethylamino)-1-methylbenzol und/oder eines seiner physiologisch verträglichen Salze und
(D) als Kuppler 5-Amino-2-methylphenol und/oder eines seiner physiologisch verträglichen Salze
   wobei
   - das molare Verhältnis aus allen im Mittel enthaltenen Entwicklern der Gruppe (A) zu allen im Mittel enthaltenen Entwicklern der Gruppe (B), d.h. das molare Verhältnis (A)/(B), bei einem Wert von 1,9 bis 2,5 liegt.

Besonders bevorzugt ist demnach ein Mittel zum oxidativen Färben von keratinischen Fasern, enthaltend in einem kosmetischen Träger
(A) als Entwickler 4,5-Diamino-1-(2-hydroxyethyl)-1H-pyrazol und/oder eines seiner physiologisch verträglichen Salze,
(B) als Entwickler 2-(2,5-Diaminophenyl)ethanol und/oder eines seiner physiologisch verträglichen Salze,
   und/oder eines seiner physiologisch verträglichen Salze,
(C) als Kuppler 2,6-Bis(2'-hydroxyethylamino)-1-methylbenzol und/oder eines seiner physiologisch verträglichen Salze und
(D) als Kuppler 5-Amino-2-methylphenol und/oder eines seiner physiologisch verträglichen Salze
   wobei
   - das molare Verhältnis aus allen im Mittel enthaltenen Entwicklern der Gruppe (A) zu allen im Mittel enthaltenen Entwicklern der Gruppe (B), d.h. das molare Verhältnis (A)/(B), bei einem Wert von 1,9 bis 2,5 liegt.

In einer weiteren Ausführungsform ist es ebenfalls bevorzugt, wenn die erfindungsgemäßen Mittel mindestens zwei Oxidationsfarbstoffvorprodukte vom Kupplertyp aus der Gruppe (C) enthalten. Bevorzugt ist auch ein Mittel zum oxidativen Färben von keratinischen Fasern, enthaltend in einem kosmetischen Träger
(A) als Entwickler 4,5-Diamino-1-(2-hydroxyethyl)-1H-pyrazol und/oder eines seiner physiologisch verträglichen Salze,
(B) als Entwickler 2-(2,5-Diaminophenyl)ethanol und/oder eines seiner physiologisch verträglichen Salze,
(C) als Kuppler 2-(2,4-Diaminophenoxy)ethanol und 2,6-Bis(2'-hydroxyethylamino)-1-methylbenzol
(D) als Kuppler mindestens ein m-Aminophenolderivat aus der Gruppe 3-Aminophenol, 5-Amino-2-methylphenol und/oder eines seiner physiologisch verträglichen Salze,
   wobei
   - das molare Verhältnis aus allen im Mittel enthaltenen Entwicklern der Gruppe (A) zu allen im Mittel enthaltenen Entwicklern der Gruppe (B), d.h. das molare Verhältnis (A)/(B), bei einem Wert von mindestens 1,2 liegt.

Bevorzugt ist auch ein Mittel zum oxidativen Färben von keratinischen Fasern, enthaltend in einem kosmetischen Träger
(A) als Entwickler 4,5-Diamino-1-(2-hydroxyethyl)-1H-pyrazol und/oder eines seiner physiologisch verträglichen Salze,
(B) als Entwickler p-Toluylendiamin und/oder eines seiner physiologisch verträglichen Salze,
(C) als Kuppler 2-(2,4-Diaminophenoxy)ethanol und 2,6-Bis(2'-hydroxyethylamino)-1-methylbenzol
(D) als Kuppler mindestens ein m-Aminophenolderivat aus der Gruppe 3-Aminophenol, 5-Amino-2-methylphenol und/oder eines seiner physiologisch verträglichen Salze,
wobei
- das molare Verhältnis aus allen im Mittel enthaltenen Entwicklern der Gruppe (A) zu allen im Mittel enthaltenen Entwicklern der Gruppe (B), d.h. das molare Verhältnis (A)/(B), bei einem Wert von mindestens 1,2 liegt.

Im Rahmen der Versuche, die für diese Anmeldung durchgeführt wurden, hat sich weiterhin herausgestellt, dass auch das molare Verhältnis der Kuppler aus den Gruppen (C) und (D) zueinander einen großen Einfluss auf die Echtheitseigenschaften nehmen kann. Die beste Waschechtheit konnte beobachtet werden, wenn die Kuppler aus der Gruppe (C) und die Kuppler aus der Gruppe (D) in ungefähr äquimolarer Menge eingesetzt wurden, d.h. wenn das Verhältnis (C)/(D) bei einem Wert bei oder in der Nähe von 1 lag. Oxidative Färbemittel, welche die Kuppler aus den Gruppen (C) und (D) in einem molaren Verhältnis von ungefähr 1 enthielten, wiesen darüber hinaus auch die besten Lichtechtheiten auf.

Die Berechnungsgrundlage für die Berechnung des molaren Verhältnisses (C)/(D) ist die molare Gesamtmenge aller im Mittel enthaltenen Kuppler der Gruppe (C) (d.h. die Molmengen von 2-(2,4-Diaminophenoxy)ethanol, 1-Methoxy-2-amino-4-(2'-hydroxyethylamino)benzol und 2,6-Bis(2'-hydroxyethylamino)-1-methylbenzol zuzüglich der Molmengen von deren Salzen), die zur molaren Gesamtmenge aller im Mittel enthaltenen Kupplern der Gruppe (D) (d.h. der Summe der Molmengen aus 3-Aminophenol und 5-Amino-2-methylphenol zuzüglich der Molmengen von deren Salzen) in Relation gesetzt wird. Hierbei gilt wieder, dass jeweils mindestens einer der vorgenannten Kuppler aus den Gruppen (C) und (D) im erfindungsgemäßen Mittel enthalten sein muss.

In einer weiteren ganz besonders bevorzugten Ausführungsform ist ein erfindungsgemäßes Mittel zum oxidativen Färben von keratinischen Fasern dadurch gekennzeichnet, dass das molare Verhältnis aus allen im Mittel enthaltenen Kupplern der Gruppe (C) zu allen im Mittel enthaltenen Kupplern der Gruppe (D), d.h. das molare Verhältnis (C)/(D), bei einem Wert von mindestens 0,5, bevorzugt von mindestens 0,6, weiter bevorzugt von mindestens 0,7 und besonders bevorzugt von mindestens 0,8 liegt.

In einer weiteren ganz besonders bevorzugten Ausführungsform ist ein erfindungsgemäßes Mittel zum oxidativen Färben von keratinischen Fasern weiterhin dadurch gekennzeichnet, dass das molare Verhältnis aus allen im Mittel enthaltenen Kupplern der Gruppe (C) zu allen im Mittel enthaltenen Kupplern der Gruppe (D), d.h. das molare Verhältnis (C)/(D), bei einem Wert von maximal 1,5, bevorzugt von maximal 1,4, weiter bevorzugt von maximal 1,3 und besonders bevorzugt von maximal 1,2 liegt.

Sowohl aus anwendungstechnischen Gründen als auch aus toxikologischen Gründen werden die Kuppler im Verhältnis zu den Entwicklern in einem geringen molaren Überschuss eingesetzt. Die besten Ergebnisse wurden erhalten, wenn das molare Verhältnis aus allen im Mittel enthaltenen Entwicklern den Gruppen (A) und (B) zu allen im Mittel enthaltenen Kupplern der Gruppen (C) und (D), d.h. das molare Verhältnis [(A)+(B)] / [(C)+(D)], bei einem Wert von ungefähr 0,75 lag, was bedeutet, dass auf 3 Molanteile Entwickler [(A) + (B)] ungefähr 4 Molanteile Kuppler [(C) + (D)] eingesetzt werden. Ähnlich gute Ergebnisse wurden auch noch innerhalb einer Schwankungsbreite erzielt, d.h. auch wenn das molare Verhältnis [(A)+(B)] / [(C)+(D)] bei Werten zwischen 0,65 und 0,80 lag, waren die Echtheiten ausgezeichnet. Darüber hinaus konnten mit Einhaltung dieser molaren Verhältnisse Hautirritationen vermieden werden.

In einer weiteren ganz besonders bevorzugten Ausführungsform ist ein erfindungsgemäßes Mittel zum oxidativen Färben von keratinischen Fasern daher dadurch gekennzeichnet, dass das molare Verhältnis aus allen im Mittel enthaltenen Entwicklern der Gruppen (A) und (B) zu allen im Mittel enthaltenen Kupplern der Gruppen (C) und (D), d.h. das molare Verhältnis [(A)+(B)] / [(C)+(D)], bei einem Wert von mindestens 0,4, bevorzugt von mindestens 0,5, weiter bevorzugt von mindestens 0,6 und besonders bevorzugt von mindestens 0,65 liegt.

In einer weiteren ganz besonders bevorzugten Ausführungsform ist ein erfindungsgemäßes Mittel zum oxidativen Färben von keratinischen Fasern weiterhin dadurch gekennzeichnet, dass das molare Verhältnis aus allen im Mittel enthaltenen Entwicklern der Gruppen (A) und (B) zu allen im Mittel enthaltenen Kupplern der Gruppen (C) und (D), d.h. das molare Verhältnis [(A)+(B)] / [(C)+(D)], bei einem Wert von maximal 0,95, bevorzugt von maximal 0,90, weiter bevorzugt von maximal 0,85 und besonders bevorzugt von maximal 0,80 liegt.

Die Berechnungsgrundlage für die Berechnung des molaren Verhältnisses [(A)+(B)] / [(C)+(D)], ist analog zu den vorgenannten Berechnungen die molare Gesamtmenge aller im Mittel enthaltenen Entwickler aus den Gruppen (A) und (B), die zu der molaren Gesamtmenge aller im Mittel enthaltenen Kuppler aus den Gruppen (C) und (D) in Relation gesetzt wird.

Das anwendungsbereite oxidative Färbemittel wird kurz vor der Anwendung durch Vermischen von zwei (oder mehr) verschiedenen Komponenten hergestellt.

Bei der ersten Komponente handelt es sich um die - bevorzugt alkalisch eingestellte - Färbezubereitung (K1), welche die Oxidationsfarbstoffvorprodukte (A), (B), (C) und (D) (sowie gegebenenfalls noch weitere zusätzliche Oxidationsfarbstoffvorprodukte und/oder weitere direktziehende Farbstoffe) enthält. Vor der Anwendung wird diese Färbezubereitung mit einer Oxidationsmittelzubereitung (K2) vermischt. Die Oxidationsmittelzubereitung (K2) ist aus Stabilitätsgründen bevorzugt auf einen sauren pH-Wert eingestellt und enthält das Oxidationsmittel. Bei dem Oxidationsmittel handelt es sich meist um Wasserstoffperoxid, welches in Form seiner wässrigen Lösung eingesetzt wird.

Die Komponenten (K1) und (K2) können in unterschiedlichen Gewichtsverhältnissen von 1:3 bis 3:1 miteinander vermengt werden und ergeben auf diese das anwendungsbereite oxidative Färbemittel. Bevorzugt werden die Komponenten (K1) und (K2) in einem Mengenverhältnis von 1:1 miteinander vermischt.

Alle Oxidationsfarbstoffvorprodukte sind in der Färbezubereitung (K1) enthalten, daher beziehen sich alle Angaben zu Gewichtsmengen, Gewichtsverhältnissen, Molmengen, Molverhältnissen und Molalitäten der Oxidationsfarbstoffvorprodukte auf die Gesamtmenge der Färbezubereitung (K1).

Es hat sich nun als vorteilhaft herausgestellt, wenn die erfindungsgemäßen Mittel zum oxidativen Färben von Keratinfasern die Entwickler der Gruppe (A) bevorzugt in einer bestimmten Gesamtmolalität enthalten.

Unter der Gesamtmolalität wird die Gesamtmolmenge aller im oxidativen Färbemittel enthaltenen Entwickler aus der Gruppe (A) verstanden, wobei sich diese Gesamtmolalität auf die Gesamtmolmenge der Entwickler (A) im Gesamtgewicht der Färbezubereitung (K1) bezieht (Einheit: mol Entwickler (A) pro kg der Färbezubereitung (K1).

In einer weiteren ganz besonders bevorzugten Ausführungsform ist ein erfindungsgemäßes Mittel zum oxidativen Färben von keratinischen Fasern daher dadurch gekennzeichnet, dass es den bzw. die Entwickler der Gruppe (A) in einer Gesamtmolalität von 0,10 bis 0,50 mol/kg, bevorzugt von 0,15 bis 0,45 mol/kg und besonders bevorzugt von 0,20 bis 0,40 mol/kg - bezogen auf das Gesamtgewicht des Mittels - enthält.

Da sich alle Oxidationsfarbstoffvorprodukte in der Färbezubereitung (K1) befinden, wird unter dem Gesamtgewicht des vorgenannten Mittels das Gesamtgewicht der Färbezubereitung (K1) verstanden. Die Färbezubereitung (K1), welche die Entwickler der Gruppe (A) besonders bevorzugt in einer Gesamtmolalität von 0,20 bis 0,40 mol/kg enthält, wird dann vor der Anwendung mit der Oxidationsmittelzubereitung (K2) vermischt. Bei einem Mischungsverhältnis (K1)/(K2) von 1:1 enthält das hierbei entstehende anwendungsbereite oxidative Färbemittel die Entwickler aus der Gruppe (A) demnach in einer Gesamtmolalität von 0,10 bis 0,20 mol/kg.

Die Kuppler der Gruppen (C) und (D) sind ebenfalls besonders bevorzugt in bestimmten Gesamtmolalitäten im erfindungsgemäßen Mittel enthalten. Bei Einsatz der Kuppler innerhalb dieser bevorzugten Mengenbereiche wurden Färbungen mit herausragenden Echtheitseigenschaften erhalten.

Unter der Gesamtmolalität wird die Gesamtmolmenge aller im oxidativen Färbemittel enthaltener Kuppler aus den Gruppen (C) und (D) verstanden, wobei sich die Gesamtmolalität auf die Gesamtmolmenge der Kuppler (C) und (D) im Gesamtgewicht der Färbezubereitung (K1) bezieht (Einheit: mol Kuppler [(C) + (D)] pro kg der Färbezubereitung (K1).

In einer weiteren ganz besonders bevorzugten Ausführungsform ist ein erfindungsgemäßes Mittel zum oxidativen Färben von keratinischen Fasern daher dadurch gekennzeichnet, dass es die Kuppler der Gruppen (C) und (D) in einer Gesamtmolalität von 0,10 bis 1,00 mol/kg, bevorzugt von 0,20 bis 0,90 mol/kg, weiter bevorzugt von 0,30 bis 0,80 mol/kg und besonders bevorzugt von 0,40 bis 0,70 mol/kg - bezogen auf das Gesamtgewicht des Mittels - enthält.

Da sich alle Oxidationsfarbstoffvorprodukte in der Färbezubereitung (K1) befinden, wird unter dem Gesamtgewicht des vorgenannten Mittels das Gesamtgewicht der Färbezubereitung (K1) verstanden. Zur weiteren Nuancierung können die erfindungsgemäßen Mittel (d.h. die Färbezubereitung (K1)) zusätzlich auch noch weitere Oxidationsfarbstoffvorprodukte vom Entwicklertyp und/oder vom Kupplertyp enthalten, die von den Entwicklern und Kupplern aus den Gruppen (A), (B), (C) und (D) verschieden sind.

Bevorzugte zusätzliche Oxidationsfarbstoffvorprodukt vom Entwickler-Typ können ausgewählt werden aus der Gruppe, die gebildet wird aus p-Phenylendiamin, 2-(1,2-Dihydroxyethyl)-p-phenylendiamin, N,N-Bis-(2-hydroxyethyl)-p-phenylendiamin, 4-Aminophenol, 4-Amino-3-methylphenol, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, 4-Amino-2-aminomethylphenol, 4-Amino-2-(1,2-dihydroxyethyl)phenol, 4-Amino-2-(diethylaminomethyl)phenol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on sowie deren physiologisch verträglichen Salzen.

Kupplerkomponenten, die zusätzlich enthalten sein können, werden bevorzugt ausgewählt aus einer der folgenden Klassen: o-Aminophenol, m-Diaminobenzol, o-Diaminobenzol und/oder deren Derivate; Naphthalinderivate mit mindestens einer Hydroxygruppe; Trihydroxybenzolderivate; Pyridinderivate; Pyrimidinderivate; Indol-Derivate und Indolin-Derivate; Pyrazolonderivate (beispielsweise 1-Phenyl-3-methylpyrazol-5-on); Morpholinderivate (beispielsweise 6-Hydroxybenzomorpholin oder 6-Aminobenzomorpholin); Chinoxalinderivate (beispielsweise 6-Methyl-1,2,3,4-tetrahydrochinoxalin), sowie Gemische aus zwei oder mehreren Verbindungen aus einer oder mehreren dieser Klassen. Bevorzugte zusätzliche m-Aminophenol-Kupplerkomponenten werden ausgewählt aus mindestens einer Verbindung aus der Gruppe Resorcin, 2-Methylresorcin, 4-Chlorresorcin, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-Diethylaminophenol, N-Cyclopentyl-3-aminophenol, 1,3-Dihydroxy-5-(methylamino)benzol, 3-Ethylamino-4-methylphenol, 2,4-Dichlor-3-aminophenol und deren physiologisch verträglichen Salzen. Bevorzugte zusätzliche m-Diaminobenzol-Kupplerkomponenten werden ausgewählt aus mindestens einer Verbindung aus der Gruppe m-Phenylendiamin, 1,3-Bis(2,4-diaminophenoxy)propan, 1,3-Bis(2,4-diaminophenyl)propan, 2-({3-[(2-Hydroxyethyl)amino]-4-methoxy-5-methylphenyl}amino)ethanol, 2-({3-[(2-Hydroxyethyl)-amino]-2-methoxy-5-methylphenyl}amino)ethanol, 2-({3-[(2-Hydroxyethyl)amino]-4,5-dimethylphenyl}-amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)aminobenzol und deren physiologisch verträglichen Salzen. Bevorzugte o-Diaminobenzol-Kupplerkomponenten werden ausgewählt aus mindestens einer Verbindung aus der Gruppe 3,4-Diaminobenzoesäure und 2,3-Diamino-1-methylbenzol und deren physiologisch verträglichen Salzen. Bevorzugte Naphthalinderivate mit mindestens einer Hydroxygruppe werden ausgewählt aus mindestens einer Verbindung der Gruppe 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin. Bevorzugte Trihydroxybenzole und deren Derivate werden ausgewählt aus mindestens einer Verbindung der Gruppe Pyrogallol und 1,2,4-Trihydroxybenzol. Bevorzugte Pyridinderivate werden ausgewählt aus mindestens einer Verbindung der Gruppe 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 3,4-Diaminopyridin, 2-(2-Methoxyethyl)amino-3-amino-6-methoxypyridin, 2-(4'-Methoxyphenyl)amino-3-aminopyridin, und deren physiologisch verträglichen Salzen. Bevorzugte Pyrimidinderivate werden ausgewählt aus mindestens einer Verbindung der Gruppe 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und deren physiologisch verträglichen Salzen. Bevorzugte Indolderivate werden ausgewählt aus mindestens einer Verbindung der Gruppe 4-Hydroxyindol, 6-Hydroxyindol und 7-Hydroxyindol und deren physiologisch verträglichen Salzen. Bevorzugte Indolinderivate werden ausgewählt aus mindestens einer Verbindung der Gruppe 4-Hydroxyindolin, 6-Hydroxyindolin und 7-Hydroxyindolin und deren physiologisch verträglichen Salzen.

Erfindungsgemäß besonders bevorzugte zusätzliche Kupplerkomponenten werden ausgewählt unter Resorcin, 2-Methylresorcin, 4-Chlorresorcin, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 1,3-Bis(2,4-diaminophenoxy)propan, 1,3-Bis-(2,4-diaminophenyl)propan, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)-amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, ,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen. Ganz besonders bevorzugt sind 5-Amino-2-methylphenol, 3-Aminophenol, 1,3-Bis(2,4-diaminophenoxy)propan, 2-Amino-3-hydroxypyridin und 1-Naphthol sowie eines deren physiologisch verträglichen Salze. Zusätzlich können die erfindungsgemäßen Mittel (d.h. die Färbezubereitung (K1)) ebenfalls mindestens einen direktziehenden Farbstoff aus der Gruppe der anionischen, nichtionischen und/oder kationischen Farbstoffe enthalten.

Besonders bevorzugt handelt es sich hierbei um einen oder mehrere nichtionische direktziehende Farbstoffe aus der Gruppe, HC Yellow 2, HC Yellow 4, HC Yellow 5, HC Yellow 6, HC Yellow 12, HC Orange 1, Disperse Orange 3, HC Red 1, HC Red 3, HC Red 7,HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 4-[(3-Hydroxypropyl)amino]-3-nitrophenol, 4-Nitro-o-phenylendiamin, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

In einer weiteren besonders bevorzugten Ausführungsform ist erfindungsgeäßes Mittel dadurch gekennzeichnet, dass es zusätzlich einen oder mehrere nichtionische direktziehende Farbstoffe aus der Gruppe HC Yellow 2, HC Yellow 4, HC Yellow 5, HC Yellow 6, HC Yellow 12, HC Orange 1, Disperse Orange 3, HC Red 1, HC Red 3, HC Red 7,HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)-amino]benzoesäure, 4-[(3-Hydroxypropyl)amino]-3-nitrophenol, 4-Nitro-o-phenylendiamin, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol enthält.

Zusätzlich können auch anionische direktziehende Farbstoffe enthalten sein, die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten sind.

Geeigenete kationische direktziehende Farbstoffe sind kationische Triphenylmethanfarbstoffe, wie beispielsweise Basic Blue 7, Basic Blue 26, Basic Violet 2 und Basic Violet 14, aromatische Systeme, die mit einer quaternären Stickstoffgruppe substituiert sind, wie beispielsweise Basic Yellow 57, Basic Red 76, Basic Blue 99, Basic Brown 16 und Basic Brown 17, kationische Anthrachinonfarbstoffe, wie HC Blue 16 (Bluequat B) sowie direktziehende Farbstoffe, die einen Heterocyclus enthalten, der mindestens ein quaternäres Stickstoffatom aufweist, insbesondere Basic Yellow 87, Basic Orange 31 und Basic Red 51. Die kationischen direktziehenden Farbstoffe, die unter dem Warenzeichen Arianor vertrieben werden, sind erfindungsgemäß ebenfalls geeignete kationische direktziehende Farbstoffe. Die zusätzlichen Oxidationsfarbstoffvorprodukte, d.h. Entwicklerkomponenten und Kupplerkomponenten, die von den Verbindungen der Gruppen (A), (B), (C) und (D) verschieden sind, sowie die optional zusätzlich enthaltenen direktziehenden Farbstoffe können beispielsweise in einer Menge von 0,0001 bis 5,0 Gew.-%, vorzugsweise 0,001 bis 3,5 Gew.-%, jeweils bezogen auf das Gesamtgewicht der Färbezubereitung (K1) verwendet werden.

Kurz vor der Anwendung wird das erfindungsgemäße Mittel (entsprechend der Färbezubereitung (K1)) mit einer Oxidationsmittelzubereitung vermischt. Auf diese Weise wird das anwendungsbereite oxidative Färbemittel erhalten.

Für eine ausreichende Quellung der Keratinfasern ist das anwendungsbereite oxidative Färbemittel bevorzugt auf einen alkalischen pH-Wert eingestellt. Auch die Färbeprozesse auf Keratinfasern laufen üblicherweise im alkalischen Milieu ab. Um die Keratinfasern und auch die Haut so weit wie möglich zu schonen, ist die Einstellung eines zu hohen pH-Wertes jedoch nicht wünschenswert. Daher ist es bevorzugt, wenn der pH-Wert des anwendungsbereiten Mittels bei einem Wert von 8,0 bis 10,5, weiter bevorzugt von 8,7 bis 10,3, noch weiter bevorzugt von 9,0 bis 10,2 und besonders bevorzugt von 9,2 bis 10,1 liegen. Bei den angegebenen pH-Werten handelt es sich um Werte, die bei einer Temperatur von 22 °C mit einer Glaselektrode gemessen wurden.

Die zur Einstellung des bevorzugten pH-Wertes erfindungsgemäß verwendbaren Alkalisierungsmittel können aus der Gruppe, die gebildet wird aus Ammoniak, Alkanolaminen, basischen Aminosäuren, sowie anorganischen Alkalisierungsmitteln wie (Erd-)Alkalimetallhydroxiden, (Erd-)-Alkalimetallmetasilikaten, (Erd-)Alkalimetallphosphaten und (Erd-)Alkalimetallhydrogenphosphaten, ausgewählt werden. Bevorzugte anorganische Alkalisierungsmittel sind Natriumhydroxid, Kaliumhydroxid, Natriumsilicat und Natriummetasilicat. Erfindungsgemäß einsetzbare, organische Alkalisierungsmittel werden bevorzugt ausgewählt aus Monoethanolamin, 2-Amino-2-methylpropanol und Triethanolamin. Die als erfindungsgemäßes Alkalisierungsmittel einsetzbaren basischen Aminosäuren werden bevorzugt ausgewählt aus der Gruppe, die gebildet wird aus Arginin, Lysin, Ornithin und Histidin, besonders bevorzugt Arginin. Es hat sich aber im Rahmen der Untersuchungen zur vorliegenden Erfindung herausgestellt, dass weiterhin erfindungsgemäß bevorzugte Mittel dadurch gekennzeichnet sind, dass sie zusätzlich ein organisches Alkalisierungsmittel enthalten. Eine Ausführungsform des ersten Erfindungsgegenstands ist dadurch gekennzeichnet, dass das Mittel zusätzlich mindestens ein Alkalisierungsmittel enthält, welches ausgewählt ist aus der Gruppe, die gebildet wird aus Ammoniak, Alkanolaminen und basischen Aminosäuren, insbesondere aus Ammoniak, Monoethanolamin und Arginin oder seinen verträglichen Salzen. Bevorzugt werden das oder die Alkalisierungsmittel zusammen mit den Oxidationsfarbstoffvorprodukten in der Färbezubereitung (K1) konfektioniert.

Ein zweiter Gegenstand der vorliegenden Erfindung ist eine Mehrkomponenten-Verpackungseinheit (Kit-of-parts) zum oxidativen Färben von keratinischen Fasern, umfassend zwei getrennt voneinander verpackte Zubereitungen (K1) und (K2), wobei
- es sich bei der Zubereitung (K1) um ein Mittel des ersten Erfindungsgegenstands handelt,
- die Zubereitung (K2) in einem wässrigen kosmetischen Träger Wasserstoffperoxid enthält, und
- die Mischung aus (K1) und (K2) einen pH-Wert von 8,0 bis 10,5, bevorzugt von 8,7 bis 10,3, weiter bevorzugt von 9,0 bis 10,2 und besonders bevorzugt von 9,2 bis 10,1 besitzt.

In einer bevorzugten Ausführungsform wird Wasserstoffperoxid selbst als wässrige Lösung in der Oxidationsmittelzubereitung (K2) verwendet. Die Konzentration einer Wasserstoffperoxid-Lösung in der Färbezubereitung (K2) wird einerseits von den gesetzlichen Vorgaben und andererseits von dem gewünschten Effekt bestimmt; vorzugsweise werden 6 bis 12 Gew.-%ige Lösungen in Wasser verwendet. Erfindungsgemäß bevorzugte Zubereitungen (K2) sind dadurch gekennzeichnet, dass sie, 5 bis 20 Gew.-%, vorzugsweise 1 bis 12,5 Gew.-%, besonders bevorzugt 2,5 bis 10 Gew.-% und insbesondere 3 bis 6 Gew.-% Wasserstoffperoxid, jeweils bezogen auf das Gesamtgewicht der Oxidationsmittelzubereitung (K2), enthalten.

Die Färbezubereitung (K1) und/oder die Oxidationsmittelzubereitung (K2) können zur Verstärkung der aufhellenden Wirkung weitere Bleichkraftverstärker enthalten, wie beispielsweise Tetraacetylethylendiamin (TAED), 1,5-Diacetyl-2,4-dioxohexahydro-1,3,5-triazin (DADHT), Tetraacetylglykoluril (TAGU), N-Nonanoylsuccinimid (NOSI), n-Nonanoyl- oder Isononanoyloxybenzolsulfonat (n- bzw. i-NOBS), Phthalsäureanhydrid, Triacetin, Ethylenglykoldiacetat und 2,5-Diacetoxy-2,5-dihydrofuran sowie Carbonatsalze bzw. Hydrogencarbonatsalze, insbesondere Ammoniumhydrogencarbonat, Ammoniumcarbonat, Natriumhydrogencarbonat, Dinatriumcarbonat, Kaliumhydrogencarbonat, Dikaliumcarbonat und Calciumcarbonat, und stickstoffhaltige, heterocyclische Bleichkraftverstärker, wie 4-Acetyl-1-methylpyridinium-p-toluolsulfonat, 2-Acetyl-1-methylpyridinium-p-toluolsulfonat, sowie N-Methyl-3,4-dihydroisochinolinium-p-toluolsulfonat.

Zur weiteren Steigerung der Aufhellung können die Färbezubereitung (K1) und/oder die Oxidationsmittelzubereitung (K2) zusätzlich mindestens eine SiO₂-Verbindung, wie Kieselsäure oder Silicate, insbesondere Wassergläser, zugesetzt sein. Die SiO2-Verbindung kann hierbei in der Färbezubereitung (K1) und/oder in der Oxidationsmittelzubereitung (K2) enthalten sein. Es kann erfindungsgemäß bevorzugt sein, die SiO₂-Verbindungen in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, jeweils bezogen auf das Gesamtgewicht der Färbezubereitung (K1) bzw. auf das Gesamtgewicht der Oxidationsmittelzubereitung (K2), einzusetzen. Die Mengenangaben geben dabei jeweils den Gehalt der SiO₂-Verbindungen (ohne deren Wasseranteil) in den Mitteln wieder.

Die oxidativen Farbänderungsmittel (d.h. die Färbezubereitung (K1) und/oder die Oxidationsmittelzubereitung (K2)) können weiterhin zusätzliche Wirk-, Hilfs- und Zusatzstoffe enthalten, um die Färbe- bzw. Aufhellleistung zu verbessern und weitere gewünschte Eigenschaften der Mittel einzustellen. Vorzugsweise werden die anwendungsbereiten Färbemittel als flüssige Zubereitung bereitgestellt und den Mitteln daher gegebenenfalls zusätzlich eine weitere oberflächenaktive Substanz zugesetzt, wobei solche oberflächenaktive Substanzen je nach Anwendungsgebiet als Tenside oder als Emulgatoren bezeichnet werden: Sie sind bevorzugt aus anionischen, zwitterionischen, amphoteren und nichtionischen Tensiden und Emulgatoren ausgewählt.

Erfindungsgemäß geeignete Mittel sind dadurch gekennzeichnet, dass das Mittel zusätzlich mindestens ein anionisches Tensid enthält. Bevorzugte anionische Tenside sind Fettsäuren, Alkylsulfate, Alkylethersulfate und Ethercarbonsäuren mit 10 bis 20 C-Atomen in der Alkylgruppe und bis zu 16 Glykolethergruppen im Molekül.

Erfindungsgemäß geeignete Mittel sind dadurch gekennzeichnet, dass das Mittel zusätzlich mindestens ein zwitterionisches Tensid enthält. Bevorzugte zwitterionische Tenside sind Betaine, N-Alkyl-N,N-dimethylammonium-glycinate, N-Acyl-aminopropyl-N,N-dimethylammoniumglycinate, und 2-Alkyl-3-carboxymethyl-3-hydroxyethyl-imidazoline. Ein bevorzugtes zwitterionisches Tensid ist unter der INCI-Bezeichnung Cocamidopropyl Betaine bekannt.

Erfindungsgemäß geeignete Mittel sind dadurch gekennzeichnet, dass das Mittel zusätzlich mindestens ein amphoteres Tensid enthält. Bevorzugte amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside sind N-Kokosalkylaminopropionat, as Kokosacylaminoethylaminopropionat und C₁₂-C₁₈-Acylsarcosin.

Weiterhin hat es sich als vorteilhaft erwiesen, wenn die Mittel weitere, nichtionogene grenzflächenaktive Stoffe, enthalten. Bevorzugte nichtionische Tenside sind Alkylpolyglykoside sowie Alkylenoxid-Anlagerungsprodukte an Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Die nichtionischen, zwitterionischen oder amphoteren Tenside werden in Anteilen von 0,1 bis 45 Gew.%, bevorzugt 1 bis 30 Gew.% und ganz besonders bevorzugt von 1 bis 15 Gew.%, bezogen auf die Gesamtmenge der anwendungsbereiten Mittel, eingesetzt.

Die anwendungsbereiten Farbveränderungsmittel können auch mindestens ein Verdickungsmittel enthalten. Bezüglich dieser Verdickungsmittel bestehen keine prinzipiellen Einschränkungen. Es können sowohl organische als auch rein anorganische Verdickungsmittel zum Einsatz kommen. Geeignete Verdickungsmittel sind anionische, synthetische Polymere, kationische, synthetische Polymere, natürlich vorkommende Verdickungsmittel, wie nichtionische Guargums, Skleroglucangums oder Xanthangums, Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, sowie Cellulosederivate, wie beispielsweise Methylcellulose, Carboxyalkylcellulosen und Hydroxyalkylcellulosen, nichtionische, vollsynthetische Polymere, wie Polyvinylalkohol oder Polyvinylpyrrolidinon; sowie anorganische Verdickungsmittel, insbesondere Schichtsilikate wie beispielsweise Bentonit, besonders Smektite, wie Montmorillonit oder Hectorit. Weiterhin hat es sich als vorteilhaft erweisen, wenn die Färbemittel, insbesondere wenn sie zusätzlich Wasserstoffperoxid enthalten, mindestens einen Stabilisator oder Komplexbildner enthalten. Besonders bevorzugte Stabilisatoren sind Phenacetin, Alkalibenzoate (Natriumbenzoat) und Salicylsäure. Weiterhin können alle Komplexbildner des Standes der Technik eingesetzt werden. Erfindungsgemäß bevorzugte Komplexbildner sind stickstoffhaltigen Polycarbonsäuren, insbesondere EDTA und EDDS, und Phosphonate, insbesondere 1-Hydroxyethan-1,1-diphosphonat (HEDP) und/oder Ethylendiamintetramethylenphosphonat (EDTMP) und/oder Diethylentriaminpentamethylenphosphonat (DTPMP) bzw. deren Natriumsalze.

Ferner können die erfindungsgemäßen Mittel weitere Wirk-, Hilfs- und Zusatzstoffe enthalten, wie beispielsweise nichtionische Polymere wie beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane; zusätzliche Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikonpolymere; kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylamino-ethylmethacrylat-Vinylpyrrolidinon-Copolymere, Vinylpyrrolidinon-Imidazolinium-methochlorid-Copolymere und quaternierter Polyvinylalkohol; zwitterionische und amphotere Polymere; anionische Polymere wie beispielsweise Polyacrylsäuren oder vernetzte Polyacrylsäuren; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; Pigmente sowie Treibmittel wie Propan-Butan-Gemische, N₂O, Dimethylether, CO₂ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in den erfindungsgemäßen Mitteln bevorzugt in Mengen von jeweils 0,0001 bis 25 Gew.%, insbesondere von 0,0005 bis 15 Gew.-%, jeweils bezogen auf das Gesamtgewicht der Färbezubereitung (K1) und/oder der Oxidationsmittelzubereitung (K2), eingesetzt.

Die erfindungsgemäßen Mittel zeigen eine außerordentlich gute Eignung zur Färbung von keratinischen Fasern, wobei die gefärbten Fasern sehr gute Waschechtheiten und sehr gute Lichtechtheiten aufweisen.

Ein weiterer Gegenstand der vorliegenden Erfindung ist daher die Verwendung eines Mittel des ersten Erfindungsgegenstands zur Verbesserung der Lichtechtheitechtheit der mit diesem Mittel erhaltenen Färbungen.

Ein weiterer Gegenstand der vorliegenden Erfindung ist die Verwendung eines Mittels des zweiten Erfindungsgegenstands zur Verbesserung der Lichtechtheitechtheit der mit diesem Mittel erhaltenen Färbungen.

Ein weiterer Gegenstand der vorliegenden Erfindung ist daher die Verwendung eines Mittel des ersten Erfindungsgegenstands zur Verbesserung der Waschechtheit der mit diesem Mittel erhaltenen Färbungen.

Ein weiterer Gegenstand der vorliegenden Erfindung ist die Verwendung eines Mittels des zweiten Erfindungsgegenstands zur Verbesserung der Waschechtheit der mit diesem Mittel erhaltenen Färbungen.

Bezüglich weiterer bevorzugter Ausführungsformen des erfindungsgemäßen Kits und der erfindungsgemäßen Verwendungen gilt mutatis mutandis das zu den erfindungsgemäßen Mitteln Gesagte.

## Patentansprüche

1. Mehrkomponenten-Verpackungseinheit (Kit-of-parts) zum oxidativen Färben von keratinischen Fasern, umfassend zwei getrennt voneinander verpackte Zubereitungen (K1) und (K2), wobei
- es sich bei der Zubereitung (K1) um ein Mittel zum oxidativen Färben von keratinischen Fasern handelt, enthaltend in einem kosmetischen Träger
(A) als Entwickler 4,5-Diamino-1-(2-hydroxyethyl)-1H-pyrazol und/oder eines seiner physiologisch verträglichen Salze,
(B) als Entwickler mindestens ein p-Diaminobenzolderivat aus der Gruppe p-Toluylendiamin, 2-(2,5-Diaminophenyl)ethanol und/oder eines seiner physiologisch verträglichen Salze,
(C) als Kuppler mindestens ein m-Diaminobenzolderivat aus der Gruppe 2-(2,4-Diaminophenoxy)-ethanol, 1-Methoxy-2-amino-4-(2'-hydroxyethylamino)benzol, 2,6-Bis(2'-hydroxyethylamino)-1-methylbenzol und/oder eines seiner physiologisch verträglichen Salze und
(D) als Kuppler mindestens ein m-Aminophenolderivat aus der Gruppe 3-Aminophenol, 5-Amino-2-methylphenol und/oder eines seiner physiologisch verträglichen Salze,
wobei
- das molare Verhältnis aus allen im Mittel (K1) enthaltenen Entwicklern der Gruppe (A) zu allen im Mittel (K1) enthaltenen Entwicklern der Gruppe (B), d.h. das molare Verhältnis (A)/(B), bei einem Wert von mindestens 1,2 liegt,
- die Zubereitung (K2) in einem wässrigen kosmetischen Träger Wasserstoffperoxid enthält, und
- die Mischung aus (K1) und (K2) einen pH-Wert von 8,0 bis 10,5 besitzt.

2. Mehrkomponenten-Verpackungseinheit (Kit-of-parts) nach Anspruch 1, **dadurch gekennzeichnet, dass** das molare Verhältnis aus allen im Mittel (K1) enthaltenen Entwicklern der Gruppe (A) zu allen im Mittel (K1) enthaltenen Entwicklern der Gruppe (B), d.h. das molare Verhältnis (A)/(B), bei einem Wert von mindestens 1,4, bevorzugt von mindestens 1,6, weiter bevorzugt von mindestens 1,8 und besonders bevorzugt von mindestens 1,9 liegt.

3. Mehrkomponenten-Verpackungseinheit (Kit-of-parts) nach einem der Ansprüche 1 oder 2, **dadurch gekennzeichnet, dass** das molare Verhältnis aus allen im Mittel (K1) enthaltenen Entwicklern der Gruppe (A) zu allen im Mittel (K1) enthaltenen Entwicklern der Gruppe (B), d.h. das molare Verhältnis (A)/(B), bei einem Wert von maximal 4,5, bevorzugt von maximal 4,0, weiter bevorzugt von maximal 3,5, noch weiter bevorzugt von maximal 3,0 und besonders bevorzugt von maximal 2,5 liegt.

4. Mehrkomponenten-Verpackungseinheit (Kit-of-parts) nach einem der Ansprüche 1 bis 3, **dadurch gekennzeichnet, dass** das Mittel (K1)
(B) als Entwickler p-Toluylendiamin und/oder eines seiner physiologisch verträglichen Salze enthält.

5. Mehrkomponenten-Verpackungseinheit (Kit-of-parts) nach einem der Ansprüche 1 bis 4, **dadurch gekennzeichnet, dass** das Mittel (K1)
(B) als Entwickler 2-(2,5-Diaminophenyl)ethanol und/oder eines seiner physiologisch verträglichen Salze enthält.

6. Mehrkomponenten-Verpackungseinheit (Kit-of-parts) nach einem der Ansprüche 1 bis 5, **dadurch gekennzeichnet, dass** das Mittel (K1)
(C) als Kuppler 2-(2,4-Diaminophenoxy)ethanol und/oder eines seiner physiologisch verträglichen Salze enthält.

7. Mehrkomponenten-Verpackungseinheit (Kit-of-parts) nach einem der Ansprüche 1 bis 6, **dadurch gekennzeichnet, dass** das Mittel (K1)
(C) als Kuppler 2,6-Bis(2'-hydroxyethylamino)-1-methylbenzol und/oder eines seiner physiologisch verträglichen Salze enthält.

8. Mehrkomponenten-Verpackungseinheit (Kit-of-parts) nach einem der Ansprüche 1 bis 7, **dadurch gekennzeichnet, dass** das Mittel (K1)
(D) als Kuppler 5-Amino-2-methylphenol und/oder eines seiner physiologisch verträglichen Salze enthält.

9. Mehrkomponenten-Verpackungseinheit (Kit-of-parts) nach einem der Ansprüche 1 bis 8, **dadurch gekennzeichnet, dass** das molare Verhältnis aus allen im Mittel (K1) enthaltenen Kupplern der Gruppe (C) zu allen im Mittel (K1) enthaltenen Kupplern der Gruppe (D), d.h. das molare Verhältnis (C)/(D), bei einem Wert von mindestens 0,5, bevorzugt von mindestens 0,6, weiter bevorzugt von mindestens 0,7 und besonders bevorzugt von mindestens 0,8 liegt.

10. Mehrkomponenten-Verpackungseinheit (Kit-of-parts) nach einem der Ansprüche 1 bis 9, **dadurch gekennzeichnet, dass** das molare Verhältnis aus allen im Mittel (K1) enthaltenen Kupplern der Gruppe (C) zu allen im Mittel (K1) enthaltenen Kupplern der Gruppe (D), d.h. das molare Verhältnis (C)/(D), bei einem Wert von maximal 1,5, bevorzugt von maximal 1,4, weiter bevorzugt von maximal 1,3 und besonders bevorzugt von maximal 1,2 liegt.

11. Mehrkomponenten-Verpackungseinheit (Kit-of-parts) nach einem der Ansprüche 1 bis 10, **dadurch gekennzeichnet, dass** das molare Verhältnis aus allen im Mittel (K1) enthaltenen Entwicklern der Gruppen (A) und (B) zu allen im Mittel (K1) enthaltenen Kupplern der Gruppen (C) und (D), d.h. das molare Verhältnis [(A)+(B)]/ [(C)+(D)], bei einem Wert von mindestens 0,4, bevorzugt von mindestens 0,5, weiter bevorzugt von mindestens 0,6 und besonders bevorzugt von mindestens 0,65 liegt.

12. Mehrkomponenten-Verpackungseinheit (Kit-of-parts) nach einem der Ansprüche 1 bis 11, **dadurch gekennzeichnet, dass** das molare Verhältnis aus allen im Mittel (K1) enthaltenen Entwicklern der Gruppen (A) und (B) zu allen im Mittel (K1) enthaltenen Kupplern der Gruppen (C) und (D), d.h. das molare Verhältnis [(A)+(B)] / [(C)+(D)], bei einem Wert von maximal 0,95, bevorzugt von maximal 0,90, weiter bevorzugt von maximal 0,85 und besonders bevorzugt von maximal 0,80 liegt.

13. Mehrkomponenten-Verpackungseinheit (Kit-of-parts) nach einem der Ansprüche 1 bis 12, **dadurch gekennzeichnet, dass** das Mittel (K1) den bzw. die Entwickler der Gruppe (A) in einer Gesamtmolalität von 0,10 bis 0,50 mol/kg, bevorzugt von 0,15 bis 0,45 mol/kg und besonders bevorzugt von 0,20 bis 0,40 mol/kg - bezogen auf das Gesamtgewicht des Mittels (K1) - enthält.

14. Mehrkomponenten-Verpackungseinheit (Kit-of-parts) nach einem der Ansprüche 1 bis 13, **dadurch gekennzeichnet, dass** das Mittel (K1) die Kuppler der Gruppen (C) und (D) in einer Gesamtmolalität von 0,10 bis 1,00 mol/kg, bevorzugt von 0,20 bis 0,90 mol/kg, weiter bevorzugt von 0,30 bis 0,80 mol/kg und besonders bevorzugt von 0,40 bis 0,70 mol/kg - bezogen auf das Gesamtgewicht des Mittels (K1) - enthält.

15. Mehrkomponenten-Verpackungseinheit (Kit-of-parts) nach einem der Ansprüche 1 bis 14, wobei
- die Mischung aus (K1) und (K2) einen pH-Wert von 8,7 bis 10,3, weiter bevorzugt von 9,0 bis 10,2 und besonders bevorzugt von 9,2 bis 10,1 besitzt.

## Claims

1. A multicomponent packaging unit (kit-of-parts) for oxidatively dyeing keratin fibers, comprising two preparations (K1) and (K2) which are separately packaged, wherein
- the preparation (K1) is an agent for oxidatively dyeing keratin fibers, containing, in a cosmetic carrier,
(A) 4,5-diamino-1-(2-hydroxyethyl)-1H-pyrazole and/or one of the physiologically acceptable salts thereof as developer,
(B) at least one p-diaminobenzene derivative from the group comprising p-toluylenediamine, 2-(2,5-diaminophenyl)ethanol and/or one of the physiologically acceptable salts thereof as developer,
(C) at least one m-diaminobenzene derivative from the group comprising 2-(2,4-diaminophenoxy)ethanol, 1-methoxy-2-amino-4-(2'-hydroxyethylamino)benzene, 2,6-bis(2'-hydroxyethylamino)-1-methylbenzene and/or one of the physiologically acceptable salts thereof as coupler, and
(D) at least one m-aminophenol derivative from the group comprising 3-aminophenol, 5-amino-2-methylphenol and/or one of the physiologically acceptable salts thereof as coupler,
wherein
- the molar ratio of all developers of group (A) contained in the agent (K1) to all developers of group (B) contained in the agent (K1), i.e. the molar ratio (A)/(B), has a value of at least 1.2,
- the preparation (K2) contains hydrogen peroxide in an aqueous cosmetic carrier, and
- the mixture of (K1) and (K2) has a pH of from 8.0 to 10.5.

2. The multicomponent packaging unit (kit-of-parts) according to claim 1, **characterized in that** the molar ratio of all developers of group (A) contained in the agent (K1) to all developers of group (B) contained in the agent (K1), i.e. the molar ratio (A)/(B), has a value of at least 1.4, preferably of at least 1.6, more preferably of at least 1.8, and particularly preferably of at least 1.9.

3. The multicomponent packaging unit (kit-of-parts) according to one of claims 1 or 2, **characterized in that** the molar ratio of all developers of group (A) contained in the agent (K1) to all developers of group (B) contained in the agent (K1), i.e. the molar ratio (A)/(B), has a value of 4.5 at most, preferably of 4.0 at most, more preferably of 3.5 at most, even more preferably of 3.0 at most, and particularly preferably of 2.5 at most.

4. The multicomponent packaging unit (kit-of-parts) according to one of claims 1 to 3, **characterized in that** the agent (K1) contains
(B) p-toluylenediamine and/or one of the physiologically acceptable salts thereof as developer.

5. The multicomponent packaging unit (kit-of-parts) according to one of claims 1 to 4, **characterized in that** the agent (K1) contains
(B) 2-(2,5-diaminophenyl)ethanol and/or one of the physiologically acceptable salts thereof as developer.

6. The multicomponent packaging unit (kit-of-parts) according to one of claims 1 to 5, **characterized in that** the agent (K1) contains
(C) 2-(2,4-diaminophenoxy)ethanol and/or one of the physiologically acceptable salts thereof as coupler.

7. The multicomponent packaging unit (kit-of-parts) according to one of claims 1 to 6, **characterized in that** the agent (K1) contains
(C) 2,6-bis(2'-hydroxyethylamino)-1-methylbenzene and/or one of the physiologically acceptable salts thereof as coupler.

8. The multicomponent packaging unit (kit-of-parts) according to one of claims 1 to 7, **characterized in that** the agent (K1) contains
(D) 5-amino-2-methylphenol and/or one of the physiologically acceptable salts thereof as coupler.

9. The multicomponent packaging unit (kit-of-parts) according to one of claims 1 to 8, **characterized in that** the molar ratio of all couplers of group (C) contained in the agent (K1) to all couplers of group (D) contained in the agent (K1), i.e. the molar ratio (C)/(D), has a value of at least 0.5, preferably of at least 0.6, more preferably of at least 0.7, and particularly preferably of at least 0.8.

10. The multicomponent packaging unit (kit-of-parts) according to one of claims 1 to 9, **characterized in that** the molar ratio of all couplers of group (C) contained in the agent (K1) to all couplers of group (D) contained in the agent (K1), i.e. the molar ratio (C)/(D), has a value of 1.5 at most, preferably of 1.4 at most, more preferably of 1.3 at most, and particularly preferably of 1.2 at most.

11. The multicomponent packaging unit (kit-of-parts) according to one of claims 1 to 10, **characterized in that** the molar ratio of all developers of groups (A) and (B) contained in the agent (K1) to all couplers of groups (C) and (D) contained in the agent (K1), i.e. the molar ratio [(A)+(B)] / [(C)+(D)], has a value of at least 0.4, preferably of at least 0.5, more preferably of at least 0.6, and particularly preferably of at least 0.65.

12. The multicomponent packaging unit (kit-of-parts) according to one of claims 1 to 11, **characterized in that** the molar ratio of all developers of groups (A) and (B) contained in the agent (K1) to all couplers of groups (C) and (D) contained in the agent (K1), i.e. the molar ratio [(A)+(B)] / [(C)+(D)], has a value of 0.95 at most, preferably of 0.90 at most, more preferably of 0.85 at most, and particularly preferably of 0.80 at most.

13. The multicomponent packaging unit (kit-of-parts) according to one of claims 1 to 12, **characterized in that** the agent (K1) contains the developer(s) of group (A) in an overall molality of from 0.10 to 0.50 mol/kg, preferably of from 0.15 to 0.45 mol/kg, and particularly preferably of from 0.20 to 0.40 mol/kg, based on the total weight of the agent (K1).

14. The multicomponent packaging unit (kit-of-parts) according to one of claims 1 to 13, **characterized in that** the agent (K1) contains the couplers of groups (C) and (D) in a total molality of from 0.10 to 1.00 mol/kg, preferably of from 0.20 to 0.90 mol/kg, more preferably of from 0.30 to 0.80 mol/kg, and particularly preferably of from 0.40 to 0.70 mol/kg, based on the total weight of the agent (K1).

15. The multicomponent packaging unit (kit-of-parts) according to one of claims 1 to 14, wherein
- the mixture of (K1) and (K2) has a pH of from 8.7 to 10.3, more preferably of from 9.0 to 10.2, and particularly preferably of from 9.2 to 10.1.

## Revendications

1. Unité de conditionnement à composants multiples (Kit-of-Parts) pour la coloration de fibres kératiniques par oxydation, comprenant deux préparations (K1) et (K2) conditionnées séparément l'une de l'autre, dans laquelle :
- il s'agit, dans la préparation (K1), d'un agent pour la coloration de fibres kératiniques par oxydation contenant, dans un support cosmétique,
(A) en tant que développeur, le 4,5-diamino-1-(2-hydroxyéthyl)-1H-pyrazole et/ou l'un de ses sels physiologiquement acceptables,
(B) en tant que développeur, au moins un dérivé de p-diaminobenzène du groupe constitué par la p-toluylènediamine, le 2-(2,5-diaminophényl)éthanol et/ou l'un de ses sels physiologiquement acceptables,
(C) en tant que coupleur, au moins un dérivé de p-diaminobenzène du groupe constitué par le 2-(2,4-diaminophénoxy)éthanol, le 1-méthoxy-2-amino-4-(2'-hydroxyéthylamino)benzène, le 2,6-bis(2'-hydroxyéthylamino)-1-méthylbenzène et/ou l'un de ses sels physiologiquement acceptables,
(D) en tant que coupleur, au moins un dérivé de m-aminophénol du groupe constitué par le 3-aminophénol, le 5-amino-2-méthylphénol et/ou l'un de ses sels physiologiquement acceptables,
dans laquelle,
- le rapport molaire de l'ensemble des développeurs du groupe (A) contenus dans l'agent (K1) à l'ensemble des développeurs du groupe (B) contenus dans l'agent (K1), à savoir, le rapport molaire (A)/(B), a une valeur d'au moins 1,2,
- la préparation (K2) contient du peroxyde d'hydrogène dans un support cosmétique aqueux, et
- le mélange de (K1) et (K2) possède une valeur de pH allant de 8,0 à 10,5.

2. Unité de conditionnement à composants multiples (Kit-of-Parts) selon la revendication 1, **caractérisée en ce que** le rapport molaire de l'ensemble des développeurs du groupe (A) contenus dans l'agent (K1) à l'ensemble des développeurs du groupe (B) contenus dans l'agent (K1), à savoir, le rapport molaire (A)/(B), a une valeur d'au moins 1,4, de préférence d'au moins 1,6, de préférence encore d'au moins 1,8 et de façon particulièrement préférée d'au moins 1,9.

3. Unité de conditionnement à composants multiples (Kit-of-Parts) selon l'une quelconque des revendications 1 ou 2, **caractérisée en ce que** le rapport molaire de l'ensemble des développeurs du groupe (A) contenus dans l'agent (K1) à l'ensemble des développeurs du groupe (B) contenus dans l'agent (K1), à savoir, le rapport molaire (A)/(B), a une valeur maximale de 4,5, de préférence de 4,0, de préférence encore de 3,5, de façon encore plus préférée de 3,0 et de façon particulièrement préférée de 2,5.

4. Unité de conditionnement à composants multiples (Kit-of-Parts) selon l'une quelconque des revendications 1 à 3, **caractérisée en ce que** l'agent (K1) contient
(B) en tant que développeur, de la p-toluylènediamine et/ou l'un de ses sels physiologiquement acceptables.

5. Unité de conditionnement à composants multiples (Kit-of-Parts) selon l'une quelconque des revendications 1 à 4, **caractérisée en ce que** l'agent (K1) contient
(B) en tant que développeur, du 2-(2,5-diaminophényl)éthanol et/ou l'un de ses sels physiologiquement acceptables.

6. Unité de conditionnement à composants multiples (Kit-of-Parts) selon l'une quelconque des revendications 1 à 5, **caractérisée en ce que** l'agent (K1) contient
(C) en tant que coupleur, du 2-(2,4-diaminophénoxy)éthanol et/ou l'un de ses sels physiologiquement acceptables.

7. Unité de conditionnement à composants multiples (Kit-of-Parts) selon l'une quelconque des revendications 1 à 6, **caractérisée en ce que** l'agent (K1) contient
(C) en tant que coupleur, du 2,6-bis(2'-hydroxyéthylamino)-1-méthylbenzène et/ou l'un de ses sels physiologiquement acceptables.

8. Unité de conditionnement à composants multiples (Kit-of-Parts) selon l'une quelconque des revendications 1 à 7, **caractérisée en ce que** l'agent (K1)
(D) contient, en tant que coupleur, du 5-amino-2-méthylphénol et/ou l'un de ses sels physiologiquement acceptables.

9. Unité de conditionnement à composants multiples (Kit-of-Parts) selon l'une quelconque des revendications 1 à 8, **caractérisée en ce que** le rapport molaire de l'ensemble des coupleurs du groupe (C) contenus dans l'agent (K1) à l'ensemble des coupleurs du groupe (D) contenus dans l'agent (K1), à savoir, le rapport molaire (C)/(D), a une valeur d'au moins 0,5, de préférence d'au moins 0,6, de préférence encore d'au moins 0,7 et de façon particulièrement préférée d'au moins 0,8.

10. Unité de conditionnement à composants multiples (Kit-of-Parts) selon l'une quelconque des revendications 1 à 9, **caractérisée en ce que** le rapport molaire de l'ensemble des coupleurs du groupe (C) contenus dans l'agent (K1) à l'ensemble des coupleurs du groupe (D) contenus dans l'agent (K1), à savoir, le rapport molaire (C)/(D), a une valeur maximale de 1,5, de préférence de 1,4, de préférence encore de 1,3 et de façon particulièrement préférée de 1,2.

11. Unité de conditionnement à composants multiples (Kit-of-Parts) selon l'une quelconque des revendications 1 à 10, **caractérisée en ce que** le rapport molaire de l'ensemble des développeurs des groupes (A) et (B) contenus dans l'agent (K1) à l'ensemble des coupleurs des groupes (C) et (D) contenus dans l'agent (K1), à savoir, le rapport molaire [(A)+(B)]/[(C)+(D)], a une valeur d'au moins 0,4, de préférence d'au moins 0,5, de préférence encore d'au moins 0,6 et de façon particulièrement préférée d'au moins 0,65.

12. Unité de conditionnement à composants multiples (Kit-of-Parts) selon l'une quelconque des revendications 1 à 11, **caractérisée en ce que** le rapport molaire de l'ensemble des développeurs des groupes (A) et (B) contenus dans l'agent (K1) à l'ensemble des coupleurs des groupes (C) et (D) contenus dans l'agent (K1), à savoir, le rapport molaire [(A)+(B)]/[(C)+(D)], a une valeur maximale de 0,95, de préférence de 0,90, de préférence encore de 0,85 et de façon particulièrement préférée de 0,80.

13. Unité de conditionnement à composants multiples (Kit-of-Parts) selon l'une quelconque des revendications 1 à 12, **caractérisée en ce que** l'agent (K1) contient le ou les développeur(s) du groupe (A) en une molarité totale de 0,10 à 0,50 mol/kg, de préférence de 0,15 à 0,45 mol/kg et de façon particulièrement préférée de 0,20 à 0,40 mol/kg - rapporté au poids total de l'agent (K1).

14. Unité de conditionnement à composants multiples (Kit-of-Parts) selon l'une quelconque des revendications 1 à 13, **caractérisée en ce que** l'agent (K1) contient les coupleurs des groupes (C) et (D) en une molarité totale allant de 0,10 à 1,00 mol/kg, de préférence de 0,20 à 0,90 mol/kg, de préférence encore de 0,30 à 0,80 mol/kg et de façon particulièrement préférée de 0,40 à 0,70 mol/kg - rapporté au poids total de l'agent (K1).

15. Unité de conditionnement à composants multiples (Kit-of-Parts) selon l'une quelconque des revendications 1 à 14, dans laquelle
- le mélange de (K1) et (K2) possède une valeur de pH allant de 8,7 à 10,3, de préférence encore de 9,0 à 10,2, et de façon particulièrement préférée de 9,2 à 10,1.
